(19) Europäisches Patentamt / European Patent Office / Office européen des brevets

(11) **EP 3 409 788 B1**

(12) **EUROPEAN PATENT SPECIFICATION**

(45) Date of publication and mention
of the grant of the patent:
**06.01.2021  Bulletin 2021/01**

(51) Int Cl.:
***C12Q 1/6869*** *(2018.01)*

(21) Application number: **17174059.0**

(22) Date of filing: **01.06.2017**

(54) **METHOD AND SYSTEM FOR NUCLEIC ACID SEQUENCING**

VERFAHREN UND SYSTEM ZUR NUKLEINSÄURESEQUENZIERUNG

PROCÉDÉ ET SYSTÈME DE SÉQUENÇAGE D'ACIDE NUCLÉIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**05.12.2018  Bulletin 2018/49**

(73) Proprietor: **Siemens Healthcare GmbH
91052 Erlangen (DE)**

(72) Inventors:
• **HEUCKMANN, Johannes
50169 Kerpen (DE)**
• **MARIOTTI, Erika
50858 Köln (DE)**

(56) References cited:
**WO-A1-2016/118883**

• **SCOTT R KENNEDY ET AL: "Detecting
ultralow-frequency mutations by Duplex
Sequencing", NATURE PROTOCOLS, vol. 9, no.
11, 9 October 2014 (2014-10-09), pages 2586-2606,
XP055390095, GB ISSN: 1754-2189, DOI:
10.1038/nprot.2014.170**

**Description**

The present invention as defined by the appended claims relates to methods and systems for nucleic acid sequencing. In particular, the present invention relates to methods and systems for reducing the number of false-positives in nucleic acid sequencing. The method comprises: aligning a plurality of genetic reads; grouping the genetic reads into a plurality of groups; creating a consensus sequence for each group of the plurality of groups by setting a representation of the most abundant nucleotide man_p or a tag N based on a ratio r between the number of genetic reads within a group of genetic reads having the most abundant nucleotide man_p at a specific position of the reference genome and the number of reads having no variant at said position; and identifying the genetic variation as a true genetic variation if a ratio r* between the number of consensus sequences comprising the tag N at a specific position p and the number of the consensus sequences comprising the genetic variation at the specific position p is below a threshold t*.
Next-generation sequencing (NGS) is a powerful technology for basic research and clinical applications because it can sequence hundreds of billions of DNA nucleotides in parallel in a single experiment. However, no sequencing technology is free of error, thus leading to false positive mutation calls. There are various sources of artificially introduced errors. For example, erroneous point mutations might be generated during PCR amplification through the incorporation of the wrong nucleotide by DNA polymerases, during cluster amplification, DNA sequencing as well as flow cell image analysis. Consequently, today's parallel sequencing technologies show error rates of about 0.5 to 1% (Shendure J, Ji H (2008) Next-generation DNA sequencing. Nat Biotechnol 26: 1135-1145; Fox EJ, et al., (2014) Accuracy of Next Generation Sequencing Platforms. Next Generat Sequenc & Applic 1: 106. doi:10.4172/jngsa.1000106). Thus, 0.5 to 1% of all nucleotides are no real variants but incorrect sequence information which is generated during sample processing. The intrinsic error-rate of NGS defines a limit below which the presence of true variants is not reliably detectable. Therefore, in standard techniques, mutations with a mutant allelic fraction below 1% cannot be detected reliably. However, the reliable detection of such mutations or single-nucleotide polymorphism (SNP) is particularly crucial in case such variations occur with a minor frequency.
Accordingly, the technical problem underlying the present invention is the provision of means and methods to provide a reliable way of nucleic acid sequencing, in particular the identification of variations in the genome.

This object is achieved with the embodiments provided herein below and with the features of the independent claims. The dependent claims relate to further aspects of the invention. The method according to the present invention may be computer-implemented. However, it is understood by the skilled person that there are also other ways of implementing the method according to the present invention.

The present invention as defined by the appended claims is related to methods and systems for nucleic acid sequencing, particularly for reducing the number of false-positives in nucleic acid sequencing. Such methods and systems of the invention can, e.g., be employed to identify (a) variation(s) in the nucleic acid sequence of a subject. The method comprises the following steps:

(a) obtaining a plurality of genetic reads by sequencing of a nucleic acid sample,
(b) aligning a plurality of genetic reads to at least one reference genetic sequence;
(c) grouping the genetic reads sharing a genetic position on a reference genetic sequence of at least one reference genetic sequence into a plurality of groups;
(d) creating a consensus sequence for each group of the plurality of groups, wherein one corresponding consensus sequence is created by determining a most abundant nucleotide *man_p* at each specific position *p* of a plurality of positions within the one corresponding group of genetic reads and

(i) setting a representation of the most abundant nucleotide *man_p* at each specific position *p* of the consensus sequence if a ratio r between the number of genetic reads within the one corresponding group having the most abundant nucleotide *man_p* at the specific position *p* and the number of genetic reads is above or equal a predetermined threshold *t*; and
(ii) setting a tag *N* if the ratio r is below the predetermined threshold *t* wherein said threshold is at least about 76%;

(e) comparing the consensus sequences of the plurality of groups to the reference genetic sequence at each specific position *p* of a plurality of positions of the consensus sequences, and wherein a difference at a specific position between the consensus sequences and the reference genetic sequence indicates a variation at the specific position;
(f) determining the number of consensus sequences comprising the variation at each specific position *p* of a plurality of positions, and determining the number of consensus sequences comprising the tag *N* at each specific position *p* of a plurality of positions; and
(g) identifying the variation at each specific position *p* of a plurality of positions as a true variation if a ratio *r** between the number of consensus sequences comprising the tag *N* at the specific position *p* and the number of the consensus sequences comprising the variation at the specific position *p* is below a threshold *t** wherein said threshold *t** is equal or below 1.8.

Alternatively, the method may comprise

(a) obtaining a plurality of genetic reads by sequencing of a nucleic acid sample, wherein the obtaining step is optionally;
(b) aligning a plurality of genetic reads to at least one reference genetic sequence;
(c) grouping the genetic reads sharing a genetic position on a reference genetic sequence into a plurality of groups;
(d) creating a consensus sequence at each specific position p of a plurality of positions for each group of the plurality of groups,
wherein a representation of a nucleotide at a specific position of the plurality of positions is set in a corresponding consensus sequence, if a ratio between the number of the nucleotide at the specific position and the number of the genetic reads within the corresponding group is above or equal a predetermined threshold t, and wherein a tag N is set in the corresponding consensus sequence at the specific position of the plurality of positions if the ratio is below the predetermined threshold t;
(e) comparing the consensus sequences of the plurality of groups to the reference genetic sequence at each specific position $p$ of a plurality of positions of the consensus sequences, and wherein a difference at a specific position between the consensus sequences and the reference genetic sequence indicates a variation at the specific position;
(f) determining the number of consensus sequences comprising the variation at each specific position $p$ of a plurality of positions, and determining the number of consensus sequences comprising the tag N at each specific position $p$ of a plurality of positions; and
(g) identifying the variation at each specific position $p$ of a plurality of positions as a true variation if a ratio $r^*$ between the number of consensus sequences comprising the tag $N$ at the specific position $p$ and the number of the consensus sequences comprising the variation at the specific position $p$ is below a threshold $t^*$.

The explanations and definitions provided in the following apply to all methods and systems provided herein mutatis mutandis. The methods and systems of the invention relate to nucleic acid sequencing. Accordingly, the method of the invention could optionally comprise obtaining a plurality of genetic reads by sequencing of a nucleic acid sample and the systems of the invention could optionally comprise an obtaining unit being configured to obtain the plurality of genetic reads of a nucleic acid sample.

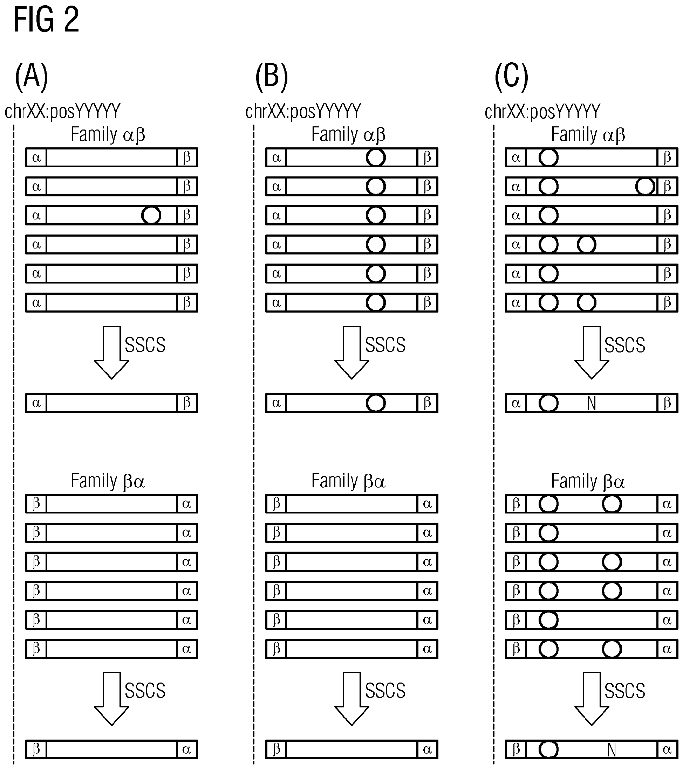

In the illustrative Example 1, it is demonstrated that the methods and systems of the invention show a high sensitivity and specificity (PPV) in detecting variations (mutations), for example, which occur in the genome with a minor allele frequency (MAF ≤ 1%). The appended examples document the analysis of genetic variations of four dilutions of three HapMap normal cell lines; see e.g. Table 1. DNA of such cell lines was analyzed by the methods and systems of the invention. The method and system provided herein include particular filtering steps that use the level of reliability for each nucleotide call. These steps are described in the following. An exemplary method is illustrated in Figures 2 to 5. In the appended examples, adaptor sequences are removed from the raw sequencing data (the genetic reads) and stored within the same file. The genetic reads are aligned to at least one reference genome. In a following step, the alignment information as well as the removed sequences, e.g. barcode sequences, are used to form groups of reads that share the same genomic position and barcode sequence. Such a group may also be designated "family". In a further step, consensus sequences are created, e.g. for each group. In the formation of consensus sequences, nucleotide for nucleotide is queried. In particular, a representation of the nucleotide at one position is only set in the consensus sequence if the nucleotide occurs with at least a particular threshold level among the reads of one group, e.g., at least 76%. This means that a representation of the most abundant nucleotide (e.g., T, A, C, G or U) at the position is only set if such a threshold is fulfilled (above or equal the threshold). If this condition is not fulfilled a tag "N" is placed at such a position. The "genetic variations" or "variations" (mutations) can be detected if a difference between the consensus sequence and the reference genome is identified. It is documented in the appended examples that such a procedure can e.g. provide a sensitivity and specificity for the detection of variations in the four dilutions of the cell lines of 86.5% and 65.5%, respectively.

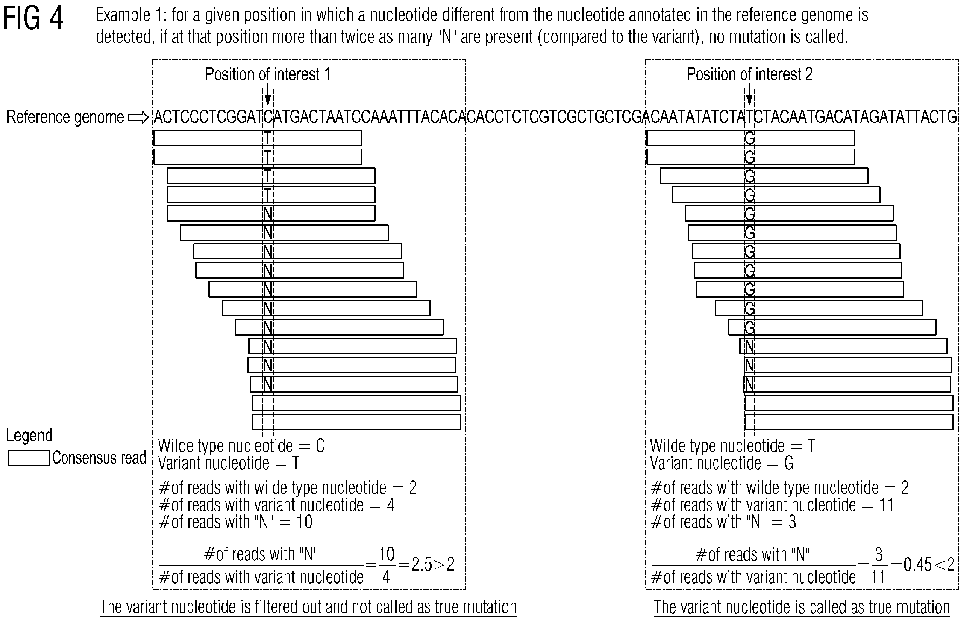

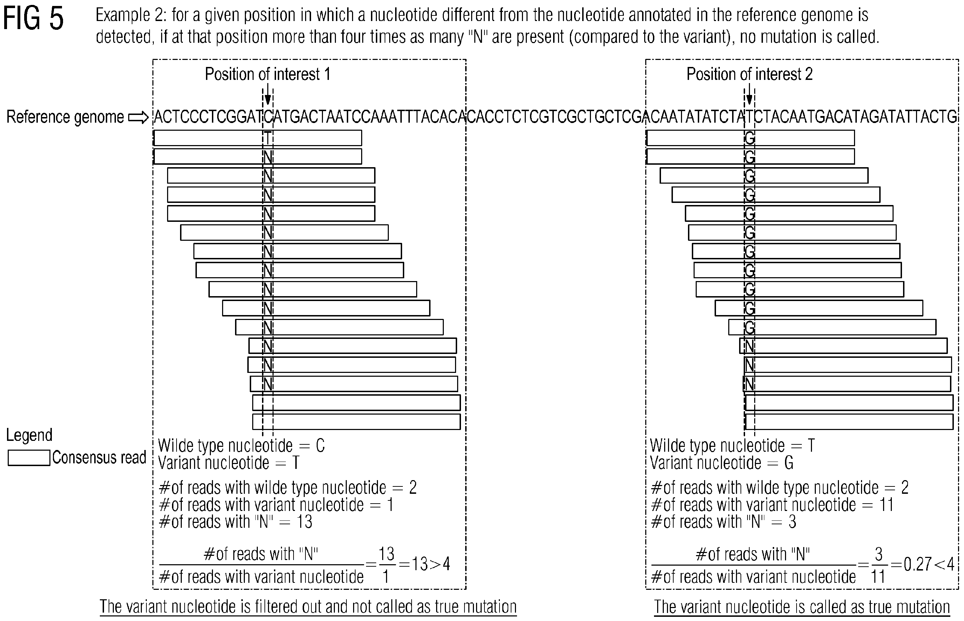

As demonstrated in the appended examples, a second filtering step ("N-Filter") was implemented in the above described method; see e.g. Figures 4 and 5. It was unexpectedly demonstrated in the appended examples that the second filtering step further improves the specificity and sensitivity for detecting genetic variations; see e.g. Table 5. In the appended Examples, it was observed that genomic regions containing a substitution erroneously introduced during the sequencing workflow exhibit a lower rate of consensus nucleotides and thus a higher number of the tag "N" compared to regions containing "true" genetic variations. Such regions are often defined by sequence repeats (e.g. Nakamura K, Sequence-specific error profile of Illumina sequencers. Nucleic Acids Res. 2011, Jul;39(13):e90. doi: 10.1093/nar/gkr344, or Schirmer M, Illumina error profiles:resolving fine-scale variation in metagenomic sequencing data. BMC Bioinformatics. 2016 Mar 11;17:125. doi: 10.1186/s12859-016-0976-y). Such repeats frequently lead to the incorporation of incorrect nucleotides, therefore increasing the likelihood of an incorporation of "N" at this position. Thus, abundancy of "N" being a surrogate for sequenced positions that do contain high background noise. Therefore, the herein provided methods and systems include the further filter that uses the abundance of "N" at a variation position to discriminate true mutation from an erroneous substitution. Accordingly, the number of consensus sequences having the genetic variation or having N at each specific position of a plurality of positions (or a specific position) in the consensus sequences is determined. Subsequently, the ratio is calculated between the number of consensus sequences containing "N" and those containing the genetic variation; see e.g. Figures 4 and 5. Accordingly, true calls can be distinguished from false positive calls. Such an unexpected effect is documented in the appended examples. This second filter is herein also referred to as the "N-Filter". In the appended Examples, the analysis of the four dilutions of the cell lines by the method including the N-filter resulted in a sensitivity and specificity of, for example, 82.5% and 80.5%, respectively; see e.g. Table 5. Accordingly, such a method allows removing a broad range of false positive variation calls. Thereby, the specificity is considerably improved. Therefore, the nucleic acid sequencing, and particularly, the identification of genetic variations (mutation calling) is improved by the methods and systems provided herein.

As indicated above, the N-filter employs a ratio between the number of consensus sequences comprising the variation at a specific position and the number of consensus sequences comprising N at the specific position. The threshold of this ratio (r*) improves specificity and sensitivity. For example, if a threshold of 2 for the ratio (r*) is applied, a sensitivity of 82.5% and specificity of 80.5% is achieved as demonstrated in the illustrative Example 1. If a threshold of 4 for the ratio (r*) is applied, a sensitivity of 87.9% and a specificity of 77.4% is provided as shown in the illustrative Example 2.

Kennedy et al., 2014 (Nature Protocols, Vol. 9 No. 11) incorporated "N" in the consensus sequence. However, the "N" information was not actively employed in the calling of the genetic variation. Therefore, such a prior art method does not include the beneficial N-Filter of the invention and is thus comparable to an analysis without employing the N-Filter. Accordingly, the methods provided herein are improved over the prior art.

In summary, the present invention has, *inter alia,* the following advantages over prior art methods and systems. The advantageous methods and systems provide sequencing of nucleic acids with a high specificity and sensitivity. Accordingly, the number of false positives in nucleic acid sequencing is reduced.
Consequently, the methods and systems provided herein provide reliable nucleic acid sequencing.

The methods and systems of the invention relate to nucleic acid sequencing. Accordingly, the methods of the invention and systems employed in such a method optionally include the step of performing nucleic acid sequencing of a nucleic acid sample. The method and systems of the invention can also be performed with obtained genetic reads. Nucleic acid sequencing is one method to obtain the plurality of genetic reads for a nucleic acid. Thus, the method of the invention can comprise nucleic acid sequencing to obtain the plurality of genetic reads for a nucleic acid comprised in a nucleic acid sample. The skilled person is aware how to perform nucleic acid sequencing, i.e., how to determine the nucleic acid sequence of a nucleic acid sample.

Accordingly, the method of invention may comprise:

(a) obtaining a plurality of genetic reads by sequencing of a nucleic acid sample;
(b) aligning the plurality of genetic reads to at least one reference genetic sequence;
(c) grouping the genetic reads sharing a genetic position on a reference genetic sequence of the at least one reference genetic sequence into a plurality of groups;
(d) creating a consensus sequence for each group of the plurality of groups, wherein one corresponding consensus sequence is created by determining a most abundant nucleotide *man_p* at each specific position *p* of a plurality of positions within the one corresponding group of genetic reads and

(i) setting a representation of the most abundant nucleotide *man_p* at each specific position *p* of the consensus sequence if a ratio r between the number genetic reads within the one corresponding group having most abundant nucleotide *man_p* at the specific position *p* and the number of genetic reads is above or equal a predetermined threshold t; and
(ii) setting a tag *N* if the ratio *r* is below the predetermined threshold *t*;

(e) comparing the consensus sequences of the plurality of groups to the reference genetic sequence at each specific position *p* of a plurality of positions of the consensus sequences, and wherein a difference at a specific position between the consensus sequences and the reference genetic sequence indicates a variation at the specific position;
(f) determining the number of consensus sequences comprising the variation at each specific position *p* of a plurality of positions, and determining the number of consensus sequences comprising the tag *N* at each specific position *p* of a plurality of positions; and
(g) identifying the variation at each specific position *p* of a plurality of positions as a true variation if a ratio *r** between the number of consensus sequences comprising the tag *N* at the specific position *p* and the number of the consensus sequences comprising the variation at the specific position $p$ is below a threshold $t^*$.

As used herein, the term "obtaining the plurality of genetic reads of a nucleic acid sample" means that the nucleic acid sequence of the nucleic acids/nucleic acid molecules comprised in the sample is determined and thereby a plurality of the genetic reads is determined.

In particular, the plurality of genetic reads is obtained or determined by sequencing of a nucleic acid sample. The nucleic acid sample as used herein comprises one or more nucleic acid molecules. Prior to obtaining a plurality of genetic reads, the nucleic acid molecules comprised in the sample could be amplified. The plurality of the genetic reads could be determined by nucleic acid sequencing.

As used herein, the term "genetic read", "read", or "sequencing read", as used herein interchangeably, or a grammatical variant thereof refers to information about a nucleic acid sequence that is obtained from a nucleic acid molecule comprised in the nucleic acid sample. The genetic read thus means a stretch of contiguous nucleotides that is a representation of a corresponding stretch in the nucleic acid sample. The genetic read may be represented symbolically by the nucleotide sequence (e.g., A, T, C, G or U). The genetic read may be obtained as the output of a nucleic acid sequencer (obtaining unit). Accordingly, the genetic reads are obtained by the nucleic acid sequencer. Thus, the genetic read can be understood as the received signal. In other words, the genetic read can be the raw sequencing data. Therefore, the genetic read may comprise an error that is artificially introduced by the obtaining unit reading the nucleic acid sample or during preparation of DNA to allow sequencing of the same. It may be stored in a memory device and processed as appropriate to determine whether it matches a reference sequence or meets other criteria. A read may be obtained directly from a sequencing apparatus or indirectly from stored sequence information concerning the sample. In some cases, a read is a sequence of sufficient length (e.g., at least about 30 nucleotides) that can be used to identify a larger sequence or region, e.g. that can be aligned and specifically assigned to a chromosome or genomic region or gene.

The plurality of the genetic reads could be determined by nucleic acid sequencing. Accordingly, the invention may particularly relate to a method comprising performing nucleic acid sequencing of one or more nucleic acid molecules comprised in a nucleic acid sample and thereby determining a plurality of genetic reads.

Prior to performing nucleic acid sequencing of a nucleic acid sample and thereby determining a plurality of genetic reads, the nucleic acid molecules may be processed. Particularly, the nucleic acid molecules may be amplified.

In nucleic acid sequencing and the subsequent alignment and grouping as provided herein, adaptor(s) may be employed. The use of adaptors is an exemplary way to facilitate the alignment and grouping. However, other means could also be used in such steps.

The (a) adaptor may be attached to the nucleic acid molecules to be sequenced and that are comprised in the nucleic acid sample. Accordingly, the methods and systems of the invention could comprise: (i) attaching at least one adaptor to a nucleic acid molecule to generate tagged nucleic acid molecules, (ii) amplifying the tagged nucleic acid molecules to produce tagged amplicons; and (iii) determining the nucleic acid sequence of the tagged amplicons, e.g. by nucleic acid sequencing. Therefore, the determination of the plurality of genetic reads and thus the nucleic acid sequencing that could be performed in the methods and systems of the invention may comprise: (i) attaching at least one adaptor to a nucleic acid molecule to generate tagged nucleic acid molecules, (ii) amplifying the tagged nucleic acid molecules to produce tagged amplicons; and (iii) determining the nucleic acid sequence of the tagged amplicons by nucleic acid sequencing and thereby determining a plurality of the genetic reads. Accordingly, the method of the invention could comprise:

(a) obtaining a plurality of genetic reads by nucleic acid sequencing of a nucleic acid sample comprising:

(i) attaching a plurality of adaptors to nucleic acid molecules comprised in the sample to generate tagged nucleic acid molecules,
(ii) amplifying the tagged nucleic acid molecules to produce tagged amplicons; and
(iii) determining the nucleic acid sequence of the tagged amplicons by nucleic acid sequencing and thereby determining a plurality of the genetic reads;

(b) aligning the plurality of genetic reads to at least one reference genetic sequence;
(c) grouping the genetic reads sharing a genetic position on a reference genetic sequence of the at least one reference genetic sequence into a plurality of groups;
(d) creating a consensus sequence for each group of the plurality of groups, wherein one corresponding consensus sequence is created by determining a most abundant nucleotide $man\_p$ at each specific position $p$ of a plurality of positions within the one corresponding group of genetic reads and

(i) setting a representation of the most abundant nucleotide $man\_p$ at each specific position $p$ of the consensus sequence if a ratio *r* between the number of genetic reads within the one corresponding group having the most abundant nucleotide *man_p* at the specific position *p* and the number of genetic reads within the one corresponding group is above or equal a predetermined threshold *t;* and

(ii) setting a tag *N* if the ratio *r* is below the predetermined threshold *t;*

(e) comparing the consensus sequences of the plurality of groups to the reference genetic sequence at each specific position *p* of a plurality of positions of the consensus sequences, and wherein a difference at a specific position between the consensus sequences and the reference genetic sequence indicates a variation at the specific position;

(f) determining the number of consensus sequences comprising the variation at each specific position *p* of a plurality of positions, and determining the number of consensus sequences comprising the tag *N* at each specific position *p* of a plurality of positions; and

(g) identifying the variation at each specific position *p* of a plurality of positions as a true variation if a ratio *r\** between the number of consensus sequences comprising the tag *N* at the specific position *p* and the number of the consensus sequences comprising the variation at the specific position *p* is below a threshold *t\**.

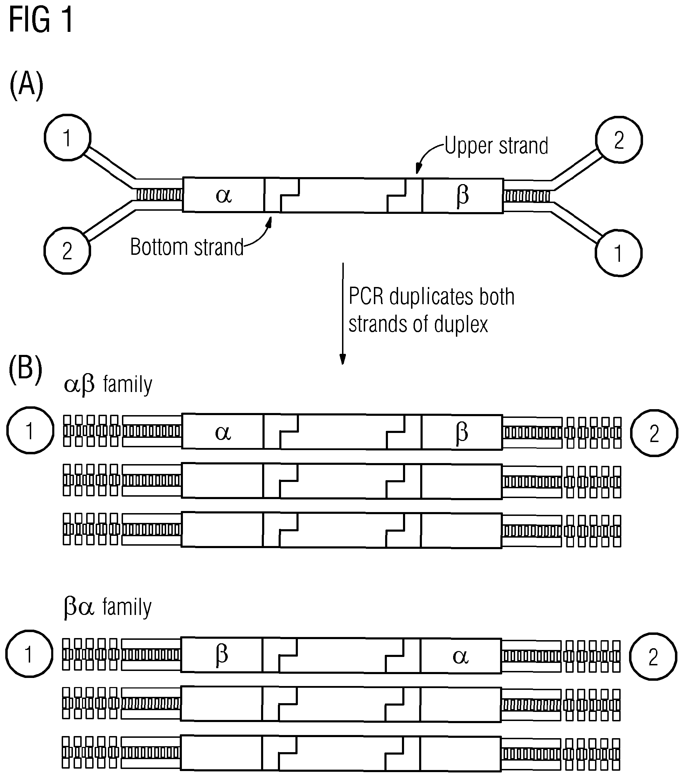

In the methods and systems of the invention, predefined adaptors may be ligated to the nucleic acid molecules. The terms "adapter" and "adaptor" are used interchangeably herein. The adaptor or adaptor molecule employed in the methods and systems of the present invention may include at least one barcode sequence. In addition, the adaptor may include a ligation adaptor. Optionally, the adaptor further includes at least one, preferably at least two PCR primer binding sites, at least one, preferably at least two sequencing primer binding sites, or both. An exemplary adaptor molecule is illustrated in Figure 1. In the methods and systems of the invention, at least one adaptor can be attached/ligated to the nucleic acid molecule. In particular, one or two adaptor(s) can be attached to a nucleic acid molecule to generate a tagged nucleic acid molecule. For example, two adaptors can be attached to the nucleic acid molecule, e.g. at either end of the nucleic acid molecule (5' and 3' end).

As used herein, attaching the adaptor to the nucleic acid molecule means that the adaptor(s) is/are ligated to the nucleic acid molecule.

The skilled person is aware of means to ligate two nucleic acid molecules together. For example, TruSeq Exome (Illumina) or SureSelectXT Reagent Kits (Agilent Technologies). As detailed below, ligation adaptors can be used to attach the adaptor(s) to the nucleic acid molecule.

A nucleic acid molecule that comprises the adaptor molecule(s), i.e., which is attached to the adaptor(s), is herein designated as "tagged nucleic acid molecule". Therefore, in order to determine the nucleic acid sequence of the nucleic acid molecules comprised in the sample, the attaching of the at least one adaptor to the nucleic acid molecule could generate tagged nucleic acid molecules.

As described above, the adaptor molecule may comprise at least one barcode sequence. Particularly, the adaptor molecule may comprise one barcode sequence. A barcode sequence is also herein referred to "single molecule identifier" (SMI) sequence. The barcode sequence means an individual sequence. In the methods provided herein, a barcode could be attached to the nucleic acid molecule comprised in the nucleic acid sample and from which the plurality of genetic reads is obtained. Accordingly, the genetic reads detected, e.g. in the nucleic acid sequencing, can comprise such barcode sequence(s) and the reads detected can thus be tagged with individual/unique nucleic acid sequence(s). Therefore, the reads can comprise the barcodes sequences in addition to their genetic information. Therefore, the genetic reads can be distinguished and sorted during data analysis based on their barcode sequence(s).

The barcode sequence as used herein can also be a single molecule identifier that naturally occurs in the nucleic acid molecule. Such a naturally occurring barcode can also be employed in the grouping of the genetic reads as described below.

The barcode sequence may be a double-stranded, complementary sequence or a single-stranded sequence. The barcode sequence can be degenerate or semi-degenerate. The degenerate or semi-degenerate barcode sequence may also be a random degenerate sequence. A double-stranded barcode sequence includes a first degenerate or semi-degenerate barcode sequence and a second barcode sequence that is complementary to the first degenerate or semi-degenerate barcode sequence, while a single-stranded barcode sequence includes a first degenerate or semi-degenerate nucleotide barcode sequence. The first and/or second degenerate or semi-degenerate barcode sequences may be any suitable length to produce a sufficiently large number of unique tags to label/tag the nucleic acid molecules of the nucleic acid sample, which may be further processed, e.g. fragmented as described above.

The barcode sequence is a short stretch of nucleotides approximately 3 to 20 nucleotides in length. Thus, the barcode sequence may be approximately 4, 5, 6, 7, 8, 9, 10, 11, 12, 13, 14, 15, 16, 17, 18, 19, 20 nucleotides in length. In principle, any length can be used as long as the information in the barcode sequence is sufficient to be distinguished and sorted during the analysis of the sequences.

The sequence of the barcode sequence may comprise any naturally occurring nucleotides, e.g., adenine (A), cytosine (C), guanine (G), thymine (T), or uracil (U), or non-natural DNA or RNA nucleotides or nucleotide-like substances or analogs with base-pairing properties (e.g., xanthosine, inosine, hypoxanthine, xanthine, 7-methylguanine, 7-methylguanosine, 5,6-dihydrouracil, 5-methylcytosine, dihydouridine, isocytosine, isoguanine, deoxynucleosides, nucleosides, peptide nucleic acids, locked nucleic acids, glycol nucleic acids and threose nucleic acids). The barcode sequences may be generated by a polymerase-mediated method or may be generated by preparing and annealing a library of individual oligonucleotides of known sequence.

As used herein, attaching a plurality of adaptors to the nucleic acid molecules means that more than one adaptor is attached to the nucleic acid molecule(s) comprised in the sample and/or that more than one different adaptors are employed in the attaching step. For example, the adaptors may differ in the barcode sequence comprised in the adaptors attached.

For example, the barcode sequence may be a random degenerate nucleotide sequence which is 12 nucleotides in length. A 12 nucleotide barcode sequence that is attached/ligated to each end of the nucleic acid molecule, as described in the Example below, results in generation of up to $4^{24}$ (i.e., $2.8 \times 10^{14}$) distinct tag sequences. In particular aspects of the invention, each nucleic acid molecules is attached or labeled with two adaptors, wherein each adaptor comprises a barcode sequence. The barcode sequences may be complementary. Such an exemplary embodiment is represented as $\alpha$ and $\beta$ in Fig.1 A that shows a schematic illustration of sheared DNA fragments labeled with double stranded predefined nucleotide sequences. Thus, in this figure, the barcodes are represented by "$\alpha$" and "$\beta$". The numbers 1 and 2 shown in Fig. 1A represent the Illumina flow-cell-compatible tails (see e.g. http://www.illumina.com/documents/products/techspotlights/tec hspotlight_sequencing.pdf for information on the Illumina flow-cell).

The adaptor can be attached/ligated to one end or to both ends of the (target) nucleic acid molecule. It is not necessary to include barcodes on both adaptor ends as long as it can be determined which strand is which.
As used herein, the "ligation adaptor" may be any suitable ligation adaptor that is complementary to a ligation adaptor added to a double-stranded target nucleic acid sequence including, but not limited to a T-overhang, an A-overhang, a CG overhang, a blunt end, or any other ligatable sequence. The ligation adaptor may be made using a method for A-tailing or T-tailing with polymerase extension; creating an overhang with a different enzyme; using a restriction enzyme to create a single or multiple nucleotide overhang, transposon based cleaving followed by adaptor tagging or any other method known in the art.

As described above, the adaptor molecule may include at least two PCR primer binding sites, such as "flow cell" binding sites. For example, a forward PCR primer binding site (or a "flow cell 1" (FC1) binding site), and a reverse PCR primer binding site (or a "flow cell 2" (FC2) binding site). The adaptor molecule may also include at least two sequencing primer binding sites, each corresponding to a sequencing read. Alternatively, the sequencing primer binding sites may be added in a separate step by inclusion of the necessary sequences as tails to the PCR primers, or by ligation of the needed sequences. Therefore, if a double-stranded target nucleic acid molecule has an SMI adaptor molecule ligated to each end, each sequenced strand will have two reads - a forward and a reverse read.

Subsequently to the step of attaching the plurality of adaptors to the nucleic acid molecules, the tagged nucleic acid molecules may be amplified. This amplification generates a set of uniquely labeled/tagged amplified nucleic acid products, i.e., amplicons. Amplification methods are known in the art (e.g., a PCR or non-PCR method).

As shown in the appended examples, the PCR amplification of the nucleic acid molecules ligated to the adaptor(s) results in two different PCR products (amplicons). Molecules obtained from the amplification of the upper strand have, for example, a barcode "$\alpha$" near the flow cell sequence number 1 (FC1) and a barcode "$\beta$" next the flow cell sequence number 2 (FC2)- referred to as "$\alpha\beta$ families" hereinafter. The amplicons resulting from the amplification of the bottom strand have the two complementary strands tagged reciprocally. Such amplicons are referred to as "$\beta\alpha$ families" hereinafter, as can also be seen in illustrative Fig.1B, which shows two different double stranded molecules resulting from the PCR amplification of the tagged nucleic acid molecules; shown in Fig. 1A. Those resulting amplicons of the upper strand have the barcode $\alpha$ near the flow cell sequence number 1 and the barcode $\beta$ near the flow cell sequence number 2, i.e., the "$\alpha\beta$ families". Amplicons resulting from the amplification of the bottom strand have the two complementary strands tagged reciprocally, referred herein as to the "$\beta\alpha$ families".

The amplified molecules, i.e., the amplicons or progeny, may then be sequenced using any suitable method known in the art. The nucleic acid sequence of the nucleic acid sample may be determined by the Illumina sequencing platform, ABI SOLiD sequencing platform, Pacific Biosciences sequencing platform, 454 Life Sciences sequencing platform, Ion Torrent sequencing platform, Helicos sequencing platform, and nanopore sequencing technology. For example, fluorescently labeled nucleotides are incorporated. The flow cell can be imaged and the emission from each cluster can be recorded. The emission wavelength and intensity can be used to identify the incorporated nucleotides. The cycle is repeated "n" times to create a genetic read length of "n" nucleotides. Accordingly, in the nucleic acid sequencing step, the genetic reads are determined. The genetic reads represent the nucleic acid sequence of the nucleic acid molecules that are comprised in the nucleic acid sample.

As used herein, "determining" or "obtaining" the genetic reads means that the nucleic acid sequence of the nucleic acid molecules is determined. A plurality of the genetic reads means that more than one genetic read is determined. The method may comprise sequencing a subset of the set of the nucleic acid molecules sufficient to produce genetic reads for at least one progeny from of each of at least 20%, at least 30%, at least 40%, at least 50%, at least 60%, at least 70%, at least 80%, at least 90% at least 95%, at least 98%, at least 99%, at least 99.9% or at least 99.99% of nucleic acid molecules comprised in the nucleic acid sample. The at least one progeny may be a plurality of progeny, e.g., at least 2, at least 5 or at least 10 progeny. As indicated above, the methods and systems of the invention can also use genetic reads that have been determined before. That is, the methods and systems of the invention can also use a determined or obtained plurality of genetic sequences.

Various adapter molecules are known in the art, e.g. WO 2013/142389. For example, an adapter molecule used in the method of the invention may form a "Y-shape" or a "hairpin shape." The "Y-shaped" adaptor allows both strands to be independently amplified by a PCR method prior to sequencing because both the top and bottom strands have binding sites for PCR primers FC1 and FC2.

Further, it is herein envisaged that artificially introduced mutations, e.g. during the amplification can be reduced by the use of different polymerases during the first round(s) of PCR. Besides polymerases, other DNA modifying/repair enzymes could be used prior to amplification to convert damage of one sort that does not give a specific mutagenic signature into another sort that does with whatever polymerase is used. Alternatively, DNA modifying/repair enzymes could be used to remove damaged bases, and one could sequence both strands of DNA both with and without the enzymatic treatment. Mutations in single-stranded DNA that are seen to be removed by the enzymatic treatment can thus be inferred to be arising due to DNA damage. This could be useful on human nuclear or mtDNA but also might also be useful with model organisms (mice, yeast, bacteria etc), treated with different new damaging agents, facilitating a screen for DNA damaging compounds that would be analogous to the widely used Ames test.

As used herein, the terms "reference genetic sequence", "reference genome" and "reference sequence" may be used interchangeably herein and refers to any particular known genome sequence, whether partial or complete, of any organism which may be used to reference identified sequences from a subject. For example, a reference genome used for human subjects as well as many other organisms is found at the National Center for Biotechnology Information at www.ncbi.nlm.nih.gov. A "genome" refers to the known genetic information of an organism.

As used herein, the terms "aligned", "alignment", or "aligning" or grammatical variants thereof refer to the process of comparing a nucleic acid sequence, e.g., a genetic read or tag, to a genetic reference sequence and thereby determining whether the reference genetic sequence contains the nucleic acid sequence, e.g., the genetic read or parts thereof. Further, if the reference genetic sequence shares genetic information with the genetic read, the read may be mapped and assigned to the particular position sharing the full or subparts of the genetic information on the reference genetic sequence. The genetic read shares genetic information with the reference genetic sequence if similarity/identity exists between the sequences.

As used herein, the term "aligning a plurality of genetic reads to at least one reference genetic sequence" means that the genetic reads that are determined in the nucleic acid sequencing or a subset thereof are aligned to the at least one reference genetic sequence. Thereby, the genetic reads are aligned to areas of similarity which may be associated with specific features that are more highly conserved than other regions occurring in the at least one genetic reference sequence. As used herein, the term "similarity" may refer to sequence identity. The areas of similarity have a specified percentage of nucleotides that are the same, when compared and aligned for maximum correspondence over a designated region (that are defined below, e.g. 72 nucleotides) as measured using a sequence comparison algorithm as known in the art, or by manual alignment and visual inspection. Sequences having, for example, 70% to 90% or greater sequence identity may be considered to be substantially identical. Particularly, the described identity/similarity exists over a stretch of at least about 10 nucleotides, preferably over a stretch of at least about 20 nucleotides, more preferably over a stretch of at least about 30 nucleotides, more preferably over a stretch of at least about 40 nucleotides, more preferably over a stretch of at least about 50 nucleotides, more preferably over a stretch of at least about 60 nucleotides, more preferably over a stretch of at least about 70 nucleotides, and most preferably over a stretch of 72 nucleotides. The similarity between the sequence of the genetic read and the reference genetic sequence may also exist of longer stretches, e.g. over a stretch of at least about 80 nucleotides, over a stretch of at least about 100 nucleotides, or over the whole length of the genetic read. Accordingly, a genetic read may be aligned to the reference genetic sequence if the genetic read is identical/similar over a stretch of at least about 5 nucleotides, preferably of at least about 10 nucleotides, more preferably over a stretch of at least about 15 nucleotides, more preferably over a stretch of at least about 20 nucleotides, more preferably over a stretch of at least about 30 nucleotides, more preferably over a stretch of at least about 40 nucleotides, more preferably over a stretch of at least about 50 nucleotides, more preferably over a stretch of at least about 60 nucleotides, more preferably over a stretch of at least about 70 nucleotides, and most preferably over a stretch of 72 nucleotides. Further, a genetic read may be aligned to the reference genetic sequence if at least about 50%, preferably at least about 60%, more preferably at least about 70%, more preferably at least about 80%, more preferably at least about 90%, more preferably at least about 95%, more preferably at least about 99%, or most preferably at least about 100% of the nucleotides are identical between the genetic read and the reference genetic sequence. Genetic reads that do not map to the reference genetic sequence according to the above defined criteria are discarded.

The alignment can be done manually, although it is typically implemented by a computer algorithm, as it would be impossible to align reads in a reasonable time period for implementing the methods disclosed herein. For example, the Efficient Local Alignment of Nucleotide Data (ELAND) computer program distributed as part of the Illumina Genomics Analysis pipeline or the Burrows-Wheeler aligner (BWA) could be used (see e.g http://bio-bwa.sourceforge.net/bwa.shtml; or Li H. and Durbin R. (2009) Fast and accurate short read alignment with Burrows-Wheeler Transform. Bioinformatics, 25:1754-60). Alternatively, a Bloom filter or similar set membership tester may be employed to align reads to reference genomes. See US Patent Application No. 61/552,374 filed October 27, 2011. In particular aspects of the invention, the plurality of genetic reads are aligned by the Burrows-Wheeler aligner (BWA).

As used herein, the term "aligning the plurality of genetic reads to at least one reference genetic sequence" means that the genetic reads, which are determined, e.g., by the nucleic acid sequencing, are aligned to the at least one reference genetic sequence. Accordingly, at least one genetic read is aligned to the at least one reference genetic sequence.

As used herein, the term "at least one reference genetic sequence" refers to at least one nucleic acid sequence (e.g. at least one genome) of a vertebrate or human. If more than one nucleic acid sequences (genomes) are meant, the aligning step can performed such that first the genetic reads are aligned to one reference genetic sequence and in the next step the genetic reads are aligned to a further reference genetic sequence, i.e., the alignment is carried out one after another. Alternatively, a consensus of more than one reference genetic sequence is created and subsequently the genetic reads are aligned to the consensus sequence of more than one reference genetic sequences. The term "at least one reference genetic sequence" may refer to one, two, three, four, five, six, seven, eight, nine, ten, fifteen, twenty or at least thirty reference genetic sequence(s).

Further, the term "at least one reference genetic sequence" could also refer to at least one nucleic acid sequence of at least one vertebrate, e.g. more than one individual of one species or one or more individual(s) of more than one species. The at least one reference genetic sequence particularly refers to at least one nucleic acid sequence of human or animal, particularly a mammal. Thus, the herein provided methods are applicable to nucleic acids of human and animals. Accordingly, the at least one reference genetic sequence may be at least one nucleic acid sequence of an animal, such as a mouse, rat, hamster, rabbit, guinea pig, ferret, cat, dog, chicken, sheep, bovine species, horse, camel, or primate. Particularly, the at least one reference genetic sequence refers to a nucleic acid sequence (genome) of one individual. Further, the at least one reference genetic sequence refers to the nucleic acid sequences (genomes) of at least one human individual. Particularly, the at least one reference genetic sequence refers to the nucleic acid sequence (genome) of one human individual or subject. The at least one reference genetic sequence can refer to a nucleic acid sequence (genome) of a healthy human subject or it can refer to (a) nucleic acid sequence(s) (genome(s)) of at least one healthy human subject, i.e., a cohort of healthy subjects. A healthy subject has no disease and/or disorder diagnosed by a physician. In certain aspects, the at least one reference genetic sequence can refer to (a) nucleic acid sequence(s) (genome(s)) of at least one human individual suffering from a disease and/or disorder. The disease and/or disorder can for example be a genetic disorder. Such a disorder may be caused by one or more abnormalities in the genome, particularly a condition that is present from birth (congenital). Genetic disorders may be hereditary, passed down from the parents' genes. Accordingly, the at least one reference genetic sequence may refer to at least one nucleic acid sequence of one or more subjects suffering from a disease and/or disorder that is selected from the group consisting of a single-gene disorder, autosomal dominant disorder, autosomal recessive disorder, X-linked dominant disorder, X-linked recessive disorder, Y-linked disorder, mitochondrial disease and a genetic disorder associated with multiple genes. The at least one reference genetic sequence may refer to (a) nucleic acid sequence(s) (genome(s)) of one or more subjects suffering from cancer and/or tumorous disease(s).

In certain aspects, the step of aligning the plurality of genetic reads to at least one reference genetic sequence means aligning the plurality of genetic reads to a single reference genetic sequence.

As used herein, the term "grouping the genetic reads sharing a genetic position on a reference genetic sequence of the at least one reference genetic sequence into a plurality of groups" means that genetic reads that align to a genetic position are grouped into a plurality of groups. As used herein, the term "genetic position" may refer to a 1 nucleotide, preferably at least 1 nucleotide, more preferably at least about 2 nucleotides, more preferably over a stretch of at least about 3 nucleotides, over a stretch of at least about 4 nucleotides stretch of at least about 5 nucleotides, preferably over a stretch of at least about 10 nucleotides, more preferably over a stretch of at least about 15 nucleotides, more preferably over a stretch of at least about 20 nucleotides, more preferably over a stretch of at least about 30 nucleotides, more preferably over a stretch of at least about 40 nucleotides, more preferably over a stretch of at least about 50 nucleotides, more preferably over a stretch of at least about 60 nucleotides, more preferably over a stretch of at least about 70 nucleotides, and most preferably over a stretch of 72 nucleotides. Therefore, genetic reads sharing the same genetic location are grouped into a plurality of groups.

As used herein, a plurality of group may refer to at least 2, 3, 5, 10, 50, 100, 1000, 100000, $1 \times 10^6$, $1 \times 10^9$ groups.

As used herein, the group size could also be at least 4, at least 5, at least 6, at least 7, at least 8, at least 9, at least 10, at least 20, at least 30, 100, at least 1000 genetic reads. Particularly, the group size may be at least three genetic reads. Accordingly, one corresponding group may comprise at least 3 genetic reads.

As used herein, the term "sharing" means that the genetic read is at least about 50%, preferably at least about 60%, more preferably at least about 70%, more preferably at least about 80%, more preferably at least about 90%, more preferably at least about 95%, more preferably at least about 99%, or most preferably at least about 100% identical to the reference genetic sequence. The genetic position can be identified by alignment of the genetic reads to each other and/or to the reference genetic sequence. Accordingly, even if the genetic reads comprise (an) erroneous nucleotide(s) that may be incorporated by the amplification of the nucleic acid sample, the grouping sorts the genetic reads such that the genetic reads are derived from the same genetic position. Accordingly, in the grouping, genetic reads that are derived from the same genetic position are sorted into groups/families. Accordingly, respective groups comprise genetic reads that are amplicons (amplification products) of the same corresponding genetic position/region.

The genetic reads are grouped sharing a genetic position on a reference genetic sequence of the at least one reference genetic sequence. Accordingly, the grouping can be based on a reference genetic sequence that is used in the aligning step. The grouping can also be based on further reference genetic sequences.

In particular aspects of the invention, the grouping step groups the genetic reads sharing a genetic position on a reference genetic sequence of the at least one reference genetic sequence into a plurality of groups and wherein each genetic read in a corresponding group of the plurality of groups comprises at least one further particular nucleic acid sequence, particularly at least one barcode sequence.

In particular, if additional sequence information, e.g. artificial sequences, such as (an) adaptor molecule(s) or barcode(s), are attached to the nucleic acid molecules, the alignment to the at least one reference genetic sequence may not consider the additional sequence information. In particular aspects, adaptor molecule(s) or barcode(s) are not aligned to the reference sequence(s). In such a step, the additional sequence information may be cut off/removed.

Accordingly, the grouping of the genetic reads can be based on (a) particular feature(s), e.g., at least one (particular) nucleic acid sequence, in the sequence of the genetic reads. In order to group the genetic reads, the genetic reads can be aligned to such (a) particular feature(s), e.g., (a) (particular) nucleic acid sequence(s). Such (a) particular feature(s) can be predetermined and/or can be selected and the genetic reads can then be aligned to such (a) feature(s). One (corresponding) group of the groups built may then comprise or consists of genetic reads that share or comprise the particular feature(s), e.g., at least one particular nucleic acid sequence. Therefore, the genetic reads of this group comprise the identical particular feature(s), e.g., identical particular nucleic acid sequences.

The particular nucleic acid sequence as used herein can be a short stretch of nucleotides, such a barcode sequence or single molecule identifier.

Accordingly, in the grouping step, the genetic reads can be aligned to the at least one reference genetic sequence and can further be aligned to at least one particular nucleic acid sequence. Such a particular nucleic acid sequence may naturally not occur in the genome of the individual of which the nucleic acid sequence is analyzed (non-natrually occuring feature). For example, such a feature can be a nucleic acid sequence that is attached to the nucleic acid molecule comprised in the nucleic acid sample. Accordingly, the at least one particular nucleic acid sequence could be attached to the nucleic acid molecule and wherein the nucleic acid molecule(s) comprised in the nucleic acid sample are analyzed by nucleic acid sequencing and thereby the plurality of genetic reads is determined. For instance, the at least one particular nucleic acid sequence may be genetic information that is comprised in the adaptor(s) attached to the nucleic acid molecule, e.g., one or more barcode sequence(s) that are comprised in the adaptor(s), as described above.

In particular aspects of the invention, the method provided herein may comprise grouping and the computation unit may be configured to group the genetic reads sharing a genetic position on a reference genetic sequence of the at least one reference genetic sequence into a plurality of groups by aligning the at least one barcode sequences, e.g., of the tagged amplicons, to each other. The grouping based on the barcode sequences provides "families" or "groups" that share the same genomic position and the identical barcode sequence(s). Accordingly, in particular aspects, the grouping of the genetic reads is an alignment to the reference genetic sequence(s) and to the barcode sequence(s). For example, the grouping may comprise the alignment of at least one particular barcode sequence. The grouping may group the genetic reads such that genetic reads of one group comprise or share one particular identical barcode sequence, or that the genetic reads of one group comprise or share at least one particular barcode sequence in one group, e.g. two individual barcode sequences. In other words, the genetic reads of one group comprises or share the identical barcode sequence(s). Thus, the genetic reads of one group may share the at least one barcode sequence. Particularly, the genetic reads of one group share two barcode sequences. Such a grouping is illustrated below in the appended examples.

In further aspects of the invention, the particular feature(s) employed in the grouping of the genetic reads may be comprised in the genome of which the genetic read is derived from. Accordingly, the particular feature(s) employed in the grouping of the genetic reads may also be at least one naturally occurring feature, e.g., (a) particular nucleic acid sequence(s) occurring in the genome of the individual of which the nucleic acid sequence is analyzed. For example, the at least one particular nucleic acid sequence corresponds to the end(s) of the nucleic acid molecules that are comprised in the nucleic acid sample. For example, randomly sheared ends (e.g. the 5' and 3' ends) of the nucleic acid molecules can be employed as particular nucleic acid sequences, e.g. as barcode sequences.

Further, the particular feature(s) employed in the grouping of the genetic reads may be a combination of at least one non-naturally occurring feature and at least one naturally occurring feature.

The genetic reads within the group could have a different length. In particular preferred aspects, the genetic reads of the group have the same length.

The invention may relate to a method for nucleic acid sequencing comprising the following steps:

(a) optionally, obtaining a plurality of genetic reads by sequencing of a nucleic acid sample;

(b) aligning the plurality of genetic reads to at least one reference genetic sequence;

(c) grouping the genetic reads sharing a genetic position on a reference genetic sequence of the at least one reference genetic sequence into a plurality of groups, wherein each genetic read in a corresponding group of the plurality of groups comprises at least one barcode sequence;

(d) creating a consensus sequence for each group of the plurality of groups, wherein one corresponding consensus sequence is created by determining the most abundant nucleotide $man\_p$ at each specific position $p$ of a plurality of positions within the one corresponding group of genetic reads and

(i) setting a representation of the most abundant nucleotide $man\_p$ at each specific position $p$ of the consensus sequence if a ratio $r$ between the number of genetic reads within the one corresponding group having the most abundant nucleotide $man\_p$ at the specific position $p$ and the number of genetic reads within the one corresponding group is above or equal a predetermined threshold $t$; and

(ii) setting a tag $N$ if the ratio $r$ is below the predetermined threshold $t$;

(e) comparing the consensus sequences of the plurality of groups to the reference genetic sequence at each specific position $p$ of a plurality of positions of the consensus sequences, and wherein a difference at a specific position between the consensus sequences and the reference genetic sequence indicates a variation at the specific position;

(f) determining the number of consensus sequences comprising the variation at each specific position $p$ of a plurality of positions, and determining the number of consensus sequences comprising the tag $N$ at each specific position $p$ of a plurality of positions; and

(g) identifying the variation at each specific position $p$ of a plurality of positions as a true variation if a ratio $r^*$ between the number of consensus sequences comprising the tag $N$ at the specific position $p$ and the number of the consensus sequences comprising the variation at the specific position $p$ is below a threshold $t^*$.

Further, the invention may relate to a method for nucleic acid sequencing comprising the following steps:

(a) obtaining a plurality of genetic reads by sequencing of a nucleic acid sample comprising:

(i) attaching a plurality of adaptors to nucleic acid molecules comprised in the sample to generate tagged nucleic acid molecules, wherein the plurality of adaptors comprises barcode sequences,

(ii) amplifying the tagged nucleic acid molecules to produce tagged amplicons; and

(iii) determining the nucleic acid sequence of the tagged amplicons by nucleic acid sequencing and thereby determining a plurality of the genetic reads; and

(b) aligning the plurality of genetic reads to at least one reference genetic sequence;

(c) grouping the genetic reads sharing a genetic position on a reference genetic sequence of the at least one reference genetic sequence into a plurality of groups, wherein each genetic read in a corresponding group of the plurality of groups comprises at least one barcode sequence;

(d) creating a consensus sequence for each group of the plurality of groups, wherein one corresponding consensus sequence is created by determining the most abundant nucleotide $man\_p$ at each specific position $p$ of a plurality of positions within the one corresponding group of genetic reads and

(i) setting a representation of the most abundant nucleotide $man\_p$ at each specific position $p$ of the consensus sequence if a ratio r between the number of genetic reads within the one corresponding group having the most abundant nucleotide $man\_p$ at the specific position $p$ and the number of genetic reads within the one corresponding group is above or equal a predetermined threshold $t$; and

(ii) setting a tag $N$ if the ratio r is below the predetermined threshold $t$;

(e) comparing the consensus sequences of the plurality of groups to the reference genetic sequence at each specific position $p$ of a plurality of positions of the consensus sequences, and wherein a difference at a specific position between the consensus sequences and the reference genetic sequence indicates a variation at the specific position;

(f) determining the number of consensus sequences comprising the variation at each specific position $p$ of a plurality of positions, and determining the number of consensus sequences comprising the tag $N$ at each specific position $p$ of a plurality of positions; and

(g) identifying the variation at each specific position $p$ of a plurality of positions as a true variation if a ratio $r^*$ between the number of consensus sequences comprising the tag $N$ at the specific position $p$ and the number of the consensus sequences comprising the variation at the specific position $p$ is below a threshold $t^*$.

Further, the order of the steps could be changed as long as the method is suitable to indentify a true genetic variant. For example, the invention may relate to a method for nucleic acid sequencing comprising the following steps:

(a) optionally, obtaining a plurality of genetic reads by sequencing of a nucleic acid sample;

(b) grouping a plurality of genetic reads into a plurality of groups, wherein the genetic reads comprised in one corresponding group share at least one particular barcode sequence,

(c) creating a consensus sequence for each group of the plurality of groups, wherein one corresponding consensus sequence is created by determining the most abundant nucleotide $man\_p$ at each specific position $p$ of a plurality of positions within the one corresponding group of genetic reads and

> (i) setting a representation of the most abundant nucleotide $man\_p$ at each specific position $p$ of the consensus sequence if a ratio $r$ between the number of genetic reads within the one corresponding group having the most abundant nucleotide $man\_p$ at the specific position $p$ and the number of genetic reads within the one corresponding group is above or equal a predetermined threshold $t$; and
> (ii) setting a tag $N$ if the ratio r is below the predetermined threshold;

(d) aligning the consensus sequences of the plurality of groups to at least one reference genetic sequence;

(e) comparing the consensus sequences of the plurality of groups to the reference genetic sequence at each specific position $p$ of a plurality of positions of the consensus sequences, and wherein a difference at a specific position between the consensus sequences and the reference genetic sequence indicates a variation at the specific position;

(f) determining the number of consensus sequences comprising the variation at each specific position $p$ of a plurality of positions, and determining the number of consensus sequences comprising the tag $N$ at each specific position $p$ of a plurality of positions; and

(g) identifying the variation at each specific position $p$ of a plurality of positions as a true variation if a ratio $r^*$ between the number of consensus sequences comprising the tag $N$ at the specific position $p$ and the number of the consensus sequences comprising the variation at the specific position $p$ is above a threshold $t^*$.

As used herein, the "corresponding group", or the "one corresponding group" refers to one group among the plurality of groups that comprises genetic reads sharing the particular feature(s), i.e., the reads of such a group comprise the identical feature(s).

As indicated above, the genetic reads are preferably grouped that share a genetic position on a reference genetic sequence of the at least one reference genetic sequence into a plurality of groups, wherein each genetic read in a corresponding group of the plurality of groups comprises at least one further particular nucleic acid sequence, such as at least one barcode sequence. This means that the genetic reads are sorted into a plurality of groups according to the genetic position (as described above) and the further particular nucleic acid sequence(s) that is/are comprised in the nucleic acid sequence of the genetic reads. Thus, the genetic reads of the one corresponding group among the plurality of groups comprise or share the same genetic position and the identical further particular nucleic sequence(s), e.g., the at least one barcode sequence. Accordingly, the genetic reads in the one corresponding group may be sorted according its genetic sequence and to at least one predetermined and/or selected nucleic acid sequence, or at least one barcode sequence. Accordingly, in particular aspects, the genetic reads in the grouping are grouped based on their genetic position and their barcode sequence.

As indicated above, a plurality of groups is formed. The plurality of groups may comprise an exemplary group', and a further exemplary group". The plurality of groups may comprise further groups, e.g., group"' to group$^n$. The genetic reads of the group" may at least partially overlap with the genetic reads of the group'. The genetic reads of the group" may not overlap with the genetic reads of the group'. The genetic reads of the group" may fully overlap with the genetic reads of the group'.

As used herein, the term "partially overlap" may mean that the genetic reads of one corresponding group (group') are identical to another group (e.g. group"), e.g. at least about 50%, at least about 60%, at least about 70%, at least about 80%, at least about 90%, at least about 95%, at least about 99%, or at least about 100% identical to the reference genetic sequence. The identity may occur at e.g. 1 nucleotide, at least 1 nucleotide, at least about 2 nucleotides, over a stretch of at least about 3 nucleotides, at least about 4 nucleotides, at least about 5 nucleotides, at least about 10 nucleotides, at least about 15 nucleotides, at least about 20 nucleotides, at least about 30 nucleotides, at least about 40 nucleotides, at least about 50 nucleotides, at least about 60 nucleotides, at least about 70 nucleotides, or at least about or equal to 72 nucleotides.

Accordingly, the grouping groups genetic reads sharing a genetic position on a reference genetic sequence of the at least one reference genetic sequence into a plurality of groups. For example, each genetic read in a corresponding group of the plurality of groups comprises at least one barcode sequence. Therefore, the genetic reads comprised in the plurality of groups share a genetic position and each group of the plurality of groups share at least one barcode sequence.

The grouping may also be performed for a second plurality of groups that shares a second genetic position on the reference genetic sequence of the at least one reference genetic sequence. Accordingly, the methods of the invention may comprise the step and the system of the invention may be configured to grouping/group the genetic reads sharing a second genetic position on the reference genetic sequence of the at least one reference genetic sequence into a second plurality of groups. Each genetic read in a corresponding group of the second plurality of groups may particularly comprise at least one barcode sequence.

The grouping may also be performed for at least a further plurality of groups that shares at least a further genetic position on the reference genetic sequence of the at least one reference genetic sequence. Accordingly, the methods of the invention may comprise the step and the system of the invention may be configured to grouping/group the genetic reads sharing at least a further genetic position on the reference genetic sequence of the at least one reference genetic sequence into at least a further plurality of groups. Each genetic read in a corresponding group of the at least a further plurality of groups may particularly comprise at least one barcode sequence.

As indicated in the examples, the complementary strand may comprise the reverse complement of the barcode sequence. The complementary strand may be identified by the reciprocal label/tag. Therefore, the genetic reads of the group' may correspond to a first strand of a double-stranded nucleic acid and the genetic reads of the group" or further group may correspond to the complementary second strand of said double-stranded nucleic acid.

The method provided herein further comprises creating and the computation unit may be configured to create a consensus sequence for each group of the plurality of groups, wherein one corresponding consensus sequence is created by determining the most abundant nucleotide *man_p* at each specific position *p* of a plurality of positions within the one corresponding group of genetic reads. Accordingly, the sequence at each specific position *p* of a plurality of positions of the genetic reads in the group or each group (or a subset thereof) is collapsed to a respective consensus sequence.

As used herein, the term "each specific position *p* of a plurality of positions" means that each position is queried of a step by step plurality of positions. For example, each position of the genetic reads within one corresponding group is queried base for base for a plurality of positions.

As used herein, the term "plurality of positions" means at least about 50%, preferably at least about 60%, more preferably at least about 70%, more preferably at least about 80%, more preferably at least about 90%, more preferably at least about 99% %, or most preferably 100% of the positions of the genetic reads or the consensus sequences are queried.

The plurality of positions within the one corresponding group of genetic reads means that the creation of the consensus sequence is performed for at least about 50%, preferably at least about 60%, more preferably at least about 70%, more preferably at least about 80%, more preferably at least about 90%, more preferably at least about 99% of the positions of the genetic reads within the group. Particularly, the creation of the consensus sequence is performed for all respective positions of the genetic reads within the corresponding group.

As used herein, the plurality of groups may refer to at least about 50%, preferably at least about 60%, more preferably at least about 70%, more preferably at least about 80%, more preferably at least about 90%, more preferably at least about 99%, or most preferably 100% of the groups, e.g. which are formed by the grouping of the genetic reads. Most preferably, all genetic reads are considered to form the consensus sequence within a group.

As used herein, each group of the plurality groups means that each group of the plurality of groups is queried, e.g. comparing, determining, or identifying, step by step. For example, the consensus sequence is created for each group that is formed in the grouping step.

In certain aspects, the method provided herein may further comprise creating and the computation unit may be configured to create a consensus sequence for each group of a plurality of groups, wherein the plurality of groups may refer to at least about 50%, preferably at least about 60%, more preferably at least about 70%, more preferably at least about 80%, more preferably at least about 90%, more preferably at least about 99%, or most preferably 100% of the groups which are formed by the grouping of the genetic reads sharing a genetic position on a reference genetic sequence.

In particular aspects, the consensus sequences are created for each group of the plurality of groups, wherein the plurality of groups correspond to 100% of the groups which are formed by the grouping of the genetic reads.

In particular aspects, the most abundant nucleotide *man_p* is determined at each specific position *p* of a plurality of positions and the representation of the most abundant nucleotide *man_p* or the tag N is set at each specific position *p* of a plurality of positions, wherein the plurality of position refer to all respective positions of the genetic reads within the corresponding group.

In the methods and systems of the invention, the consensus sequence for each group of the plurality of groups can be created by determining the most abundant nucleotide *man_p* at each specific position *p* of a plurality of positions within the one corresponding group of genetic reads and by setting a representation of the most abundant nucleotide man_p or a tag N based on a threshold t.

As used herein, the "consensus sequence" is the calculated order of most frequent nucleotides found at corresponding positions in the genetic reads that are comprised in the group, wherein the consensus sequence comprises a representation of the nucleotide (man_p) or a tag N as defined below.

The most "frequent nucleotide" or "most abundant nucleotide" at a specific position p is herein referred to as *"man_p"*. The (representation of the) most abundant nucleotide at the specific position *p* refers to either adenine ("A"), guanine ("G"), cytosine ("C"), thymine ("T") or uracil ("U") or any other nucleobase present in the nucleic acid sequence of the genetic reads. Thus, as used herein, the "representation of the most abundant nucleotide *man_p"* may refer to the predominantly occurring nucleobase adenine ("A"), guanine ("G"), cytosine ("C"), thymine ("T") or uracil ("U") in the genetic reads within one corresponding group.

The consensus sequences can be created by comparing the sequences of the genetic reads to each other, which are aligned in the groups, e.g., the genetic reads sharing the genetic position. The nucleic acid sequence of the genetic reads in a group can be scored nucleotide for nucleotide. The nucleotides can be scored and the nucleotide at a specific position p can be determined to be the "most abundant nucleotide" or "*man_p*" in the genetic reads within one corresponding group. For example, the nucleotide at a specific position p is determined to be the most abundant nucleotide and set in the consensus sequence it is occurs at this particular position p within the genetic reads of this particular group with a particular threshold (e.g. referred to as t), e.g. at least about 76%.

The representation of the most abundant nucleotide *man_p* is set in the consensus sequence if a ratio r between the number of genetic reads within the one corresponding group having the most abundant nucleotide *man_p* at the specific position *p* and the number of genetic reads within the one corresponding is above or equal a predetermined threshold *t*. If the ratio *r* is below the predetermined threshold *t*, the nucleotide at the specific position is set "*N*". Thus, the ratio *r* determines whether the representation of the most abundant nucleotide is set or whether the tag "N" is set in the consensus sequence. The ratio r is calculated between the number of genetic reads within the one corresponding group having the most abundant nucleotide *man_p* at the specific position *p* and the number of genetic reads within the one corresponding group.

Accordingly, the term of the methods and systems provided herein of "creating a consensus sequence for each group of the plurality of groups" can refer to creating a consensus sequence for each group of the plurality of groups, wherein one corresponding consensus sequence is created by setting a representation of the most abundant nucleotide *man_p* at each specific position *p* of a plurality of positions of the consensus sequence if a ratio *r* between the number of genetic reads within the one corresponding group having the most abundant nucleotide *man_p* at the specific position *p* and the number of genetic reads within the one corresponding group is above or equal a predetermined threshold *t;* and setting a tag *N* if the ratio *r* is below the predetermined threshold *t* .

Accordingly in the methods and systems provided herein, the consensus sequence at each specific position *p* of a plurality of positions within the group of genetic reads may be created, wherein a representation of the most abundant nucleotide is set in the consensus sequence if this nucleotide occurs at this specific position with a particular occurrence, wherein the occurrence is the threshold t, and wherein a tag N is set in the consensus sequence if this nucleotide does not occur at this specific position with the particular occurrence.

As used herein, the term the "number of genetic reads" refers to the number of the genetic reads which are comprised in one corresponding group of the plurality of groups. For example, this term may refer to the number of genetic reads within one group of the plurality of groups, wherein in this one group the genetic reads share a genetic position and optionally share at least one barcode sequence.

As used herein, the term the "number of genetic reads within the one corresponding group having the most abundant nucleotide *man_p* at the specific position *p"* refers to the number of the genetic reads, that are comprised in one corresponding group of the plurality of groups, and that have the most abundant nucleotide *man_p* at a particular position *p* of the plurality of positions. Thus, it refers to the number of genetic reads within the group that have at this particular position an identical most abundant nucleotide.

The invention comprises in the methods and systems creating a consensus sequence by determining the most abundant nucleotide *man_p* at each specific position *p* of a plurality of positions within the one corresponding group of genetic reads and

(i) setting a representation of the most abundant nucleotide *man_p* at each specific position *p* of the consensus sequence if a ratio *r* between the number of genetic reads within the one corresponding group having the most abundant nucleotide *man_p* at the specific position *p* and the number of genetic reads within the one corresponding group is above or equal a predetermined threshold t; and

(ii) setting a tag *N* if the ratio r between the number of genetic reads within the one corresponding group having the most abundant nucleotide *man_p* at the specific position *p* and the number of genetic reads within the one corresponding group is below the predetermined threshold *t*.

In other words, the methods and systems provided herein create a consensus sequence at each specific position p of a plurality of positions for each group of the plurality of groups,

wherein a representation of a nucleotide at one specific position of the plurality of positions is set in a corresponding consensus sequence, if a ratio between the number of the nucleotide at the specific position and the number of the genetic reads within the corresponding group is above or equal a predetermined threshold t, and

wherein a tag N is set in the corresponding consensus sequence at the specific position of the plurality of positions if the ratio is below the predetermined threshold t.

In particular aspects, the methods and systems provided herein create a consensus sequence at each specific position p of a plurality of positions for each group of the plurality of groups,

wherein the nucleotide(s) are determined at each of the specific position of the plurality of positions, and

wherein a representation of a nucleotide of the nucleotide(s) at one specific position of the plurality of positions is set in a corresponding consensus sequence, if a ratio between the number of this nucleotide at the specific position and the number of the genetic reads within the corresponding group is above or equal a predetermined threshold t, and

wherein a tag N is set in the corresponding consensus sequence at this specific position if the ratio is below the predetermined threshold t.

Accordingly, in order to create the consensus sequence, it is not required to determine the most abundant nucleotide man_p as long as the nucleotide of which the representation is set in the consensus sequence fulfils the threshold t, i.e., the particular value of the ratio r.

As used herein, the threshold "*t*" or "t" refers to any number that is used as a cutoff. The threshold t can be identified by analyzing training sets. The threshold herein refers to a value in percent. Thus, the value determined by the ratio r can be multiplied by 100 in order to obtain a value that corresponds to the value in %. As described in the appended examples, established samples, e.g. cell lines, such as the HapMap normal cell lines (e.g. as given in Table 1), can be used to determine an appropriate threshold value. The genome sequence of such cell lines is known. Therefore, the appropriate threshold t can be determined that results in the most favorable result. In particular, the following procedure can be used to identify an appropriate threshold t:

With a sequencing error of 1%, the likelihood of accumulating an incorrect base at the same position is 0.01 (1% error) / 3 (e.g. 3 possible incorrect bases if A is correct, G, T and C may be artifacts) for each individual read. See illustrative table below for different exemplary family sizes (e.g. 3 - 6) and the respective likelihoods of recurrent sequencing errors (annotated with an individual likelihood of 0.0033).

| read 1 | read 2 | read 3 | read 4 | read 5 | read 6 | cutoff | cutoff | cutoff | propability | propability in 1 of 10 000 reads | propability in % |
|---|---|---|---|---|---|---|---|---|---|---|---|
| 0.0033 | 0.0033 | 0.0033 | | | | **YES** | **YES** | **YES** | 3.5937E-08 | 0.00035937 | 0.035937 |
| 0.0033 | 0.0033 | 0.0033 | 0.99 | | | **YES** | **YES** | NO | 3.55776 | 0.00035 | 0.03557 |

| 33 | 33 | 33 | | | | S | S | | E-08 | 5776 | 763 |
|---|---|---|---|---|---|---|---|---|---|---|---|
| 0.00 33 | 0.00 33 | 0.00 33 | 0.99 | 0. 99 | | YE S | NO | NO | 3.52219 E-08 | 0.00035 2219 | 0.03522 1854 |
| 0.00 33 | 0.00 33 | 0.00 33 | 0.99 | 0. 99 | 0. 99 | NO | NO | NO | 3.48696 E-08 | 0.00034 8696 | 0.03486 9635 |
| 0.00 33 | 0.00 33 | 0.00 33 | 0.00 33 | 0. 99 | | YE S | YE S | YE S | 1.17406 E-10 | 1.17406 E-06 | 0.00011 7406 |
| 0.00 33 | 0.00 33 | 0.00 33 | 0.00 33 | 0. 99 | 0. 99 | YE S | NO | NO | 1.16232 E-10 | 1.16232 E-06 | 0.00011 6232 |

As indicated in the table above, depending on the cutoff chosen, the likelihood of artifacts being considered as true mutations may vary.

Thus, if the threshold is defined as 76%, at least 4 incorrect and identical bases could occur at the particular position with a likelihood of (0.0033*0.0033*0.0033*0.0033) 1.2E-10). Lower thresholds may increase the respective likelihood accordingly. As a consequence, lower thresholds can lead to consensus sequences that contain sequencing errors and therefore can lead to artificial mutation calls. This risk is increased in a setting in which the same region of interested is sequenced with a sequencing depth of several thousand. For example, at a sequencing depth of 10 000X, a cutoff of 75% or greater would lead to a likelihood of

0.0033*0.0033*0.0033*0.99 = 3.55776E-08 * 10 OOOX = 0.000355776 or ~ 0.04% of an artificial consensus in a read family with 4 members. Considering the overall bases that might be sequenced in the respective assay, this likelihood may be considered too high. Conversely, unless all three reads of an exemplary family of 3 contain sequencing artifacts, a cutoff of 76% leads to a likelihood of 0.0033*0.0033*0.0033*0.0033= 1.18592E-10 * 10 000X = 1.18592E-06 or ~ 0.0001% of an artificial consensus.

The choice of the threshold is dependent on the level of confidence that the user wishes to have to make the classification.

It may advantageous to use larger sets of qualified samples to improve the utility of the threshold values.

In particular aspects, the predetermined threshold t is about 76%.

The predetermined threshold $t$ may be about 99%, preferably the predetermined threshold $t$ is about 95%, more preferably the predetermined threshold $t$ is about 90%, more preferably the predetermined threshold $t$ is about 85%, more preferably the predetermined threshold $t$ is about 80%, more preferably the predetermined threshold $t$ is about 79%, more preferably the predetermined threshold $t$ is about 78%, more preferably the predetermined threshold $t$ is about 77%, or most preferably the predetermined threshold t is about 76%.

Without being bound by theory, an exemplary group size of between 3 and 8 genetic reads can be used to gain an optimal ratio of "consensus" and "sequencing costs". Accordingly, the skilled person can determine how many reads within a group, e.g. of 3, 4, 5, 6, 7 or 8 genetic reads, may be needed to form a consensus sequence. As shown in the appended examples, 4 out of 5 genetic reads improved the analysis. 76% represent the situation in which 4 out of 5 genetic reads within a group share the same nucleotide sequence at a given position. Two out of three, three out of four genetic reads may also be employed.

The method and system provided herein comprises in creating the consensus sequence setting the representation of the most abundant nucleotide $man\_p$ at each specific position $p$ of the consensus sequence if a ratio r between the number of genetic reads within the one corresponding group having the most abundant nucleotide $man\_p$ at the specific position $p$ and the number of genetic reads within the one corresponding group is above or equal about 55%, and setting a tag $N$ if the ratio is below about 55%.

Preferably, the method and system comprise in creating the consensus sequence setting the representation of the most abundant nucleotide $man\_p$ at each specific position $p$ of the consensus sequence if a ratio $r$ is above or equal about 60%, and setting a tag $N$ if the ratio $r$ is below about 60%.

More preferably, the method and system comprise in creating the consensus sequence setting the representation of the most abundant nucleotide $man\_p$ at each specific position $p$ of the consensus sequence if a ratio $r$ is above or equal about 65%, and setting a tag N if the ratio r is below about 65%.

More preferably, the method and system comprise in creating the consensus sequence setting the representation of the most abundant nucleotide $man\_p$ at each specific position $p$ of the consensus sequence if a ratio $r$ is above or equal about 68%, and setting a tag N if the ratio r is below about 68%.

More preferably, the method and system comprise in creating the consensus sequence setting the representation of the most abundant nucleotide *man_p* at each specific position *p* of the consensus sequence if a ratio r is above or equal about 70%, and setting a tag N if the ratio r is below about 70%.

More preferably, the method and system comprise in creating the consensus sequence setting the representation of the most abundant nucleotide *man_p* at each specific position *p* of the consensus sequence if a ratio *r* is above or equal about 71%, and setting a tag *N* if the ratio r is below about 71%.

More preferably, the method and system comprise in creating the consensus sequence setting the representation of the most abundant nucleotide *man_p* at each specific position *p* of the consensus sequence if a ratio *r* is above or equal about 72%, and setting a tag *N* if the ratio *r* is below about 72%.

More preferably, the method and system comprise in creating the consensus sequence setting the representation of the most abundant nucleotide *man_p* at each specific position *p* of the consensus sequence if a ratio *r* is above or equal about 73%, and setting a tag *N* if the ratio is below about 73%.

More preferably, the method and system comprise in creating the consensus sequence setting the representation of the most abundant nucleotide *man_p* at each specific position *p* of the consensus sequence if a ratio *r* is above or equal about 74%, and setting a tag *N* if the ratio *r* is below about 74%.

More preferably, the method and system comprise in creating the consensus sequence setting the representation of the most abundant nucleotide *man_p* at each specific position *p* of the consensus sequence if a ratio *r* is above or equal about 75%, and setting a tag *N* if the ratio *r* is below about 75%.

More preferably, the method and system comprise in creating the consensus sequence setting the representation of the most abundant nucleotide *man_p* at each specific position *p* of the consensus sequence if a ratio *r* is above or equal about 76%, and setting a tag *N* if the ratio r is below about 76%.

Further, the method and system comprise in creating the consensus sequence setting the representation of the most abundant nucleotide *man_p* at each specific position *p* of the consensus sequence if a ratio *r* is above or equal about 99%, and setting a tag *N* if the ratio *r* is below about 99%.

Preferably, the method and system comprise in creating the consensus sequence setting the representation of the most abundant nucleotide *man_p* at each specific position *p* of the consensus sequence if a ratio *r* is above or equal about 95%, and setting a tag *N* if the ratio r is below about 95%.

More preferably, the method and system comprise in creating the consensus sequence setting the representation of the most abundant nucleotide *man_p* at each specific position *p* of the consensus sequence if a ratio is above or equal about 90%, and setting a tag *N* if the ratio r is below about 90%.

More preferably, the method and system comprise in creating the consensus sequence setting the representation of the most abundant nucleotide *man_p* at each specific position *p* of the consensus sequence if a ratio *r* is above or equal about 85%, and setting a tag *N* if the ratio is below about 85%.

More preferably, the method and system comprise in creating the consensus sequence setting the representation of the most abundant nucleotide *man_p* at each specific position *p* of the consensus sequence if a ratio *r* is above or equal about 80%, and setting a tag N if the ratio *r* is below about 80%.

More preferably, the method and system comprise in creating the consensus sequence setting the representation of the most abundant nucleotide *man_p* at each specific position *p* of the consensus sequence if a ratio *r* is above or equal about 79%, and setting a tag *N* if the ratio *r* is below about 79%.

More preferably, the method comprises in creating the consensus sequence setting the representation of the most abundant nucleotide *man_p* at each specific position *p* of the consensus sequence if a ratio *r* is above or equal about 78%, and setting a tag N if the ratio *r* is below about 78%.

More preferably, the method and system comprise in creating the consensus sequence setting the representation of the most abundant nucleotide *man_p* at each specific position *p* of the consensus sequence if a ratio *r* is above or equal about 77%, and setting a tag *N* if the ratio *r* is below about 77%.

In certain aspects, creating the consensus sequence can also be performed for each group of the second plurality of groups. In certain aspects, creating the consensus sequence can also be performed for each group of at least a further plurality of groups or one second plurality of groups.

The creation of a consensus sequence of a second plurality of groups or at least one further plurality of groups may involve a further aligning of the plurality of genetic reads to at least one reference genetic sequence and/or grouping the genetic reads.

As described above, the genetic reads of the group' of the plurality of groups may correspond to a first strand of a double-stranded nucleic acid and the genetic reads of the group" may correspond to the complementary second strand of said double-stranded nucleic acid. The methods and systems of the invention may perform the step creating a consensus sequence as defined herein for at least one second group or a further group.

The exemplary group" or further group may correspond to the complementary second strand, thus the consensus sequence of a double stranded nucleic acid may be created. Such a consensus sequence is herein also referred to double-stranded consensus sequence or duplex consensus sequences. The corresponding double-stranded consensus sequence may be created by determining the most abundant nucleotide *man_p* at each specific position *p* of a plurality of positions within the genetic reads of the corresponding group' and group" of, and wherein each position corresponds to a base pair.

The double-stranded consensus sequence could be created by several procedures, not limited to those provided in the following.

The method for nucleic acid sequencing, particularly for reducing the number of false-positives in nucleic acid sequencing, may comprise the following steps:

(a) (optionally obtaining a plurality of genetic reads by sequencing of a nucleic acid sample;)

(b) aligning the plurality of genetic reads to at least one reference genetic sequence;

(c) grouping the genetic reads sharing a genetic position on a reference genetic sequence of the at least one reference genetic sequence into a plurality of groups;

(d) creating a consensus sequence for each group of the plurality of groups, wherein one corresponding consensus sequence is created by determining the most abundant nucleotide *man_p* at each specific position *p* of a plurality of positions within the one corresponding group of genetic reads and

(i) setting a representation of the most abundant nucleotide *man_p* at each specific position *p* of the consensus sequence if a ratio r between the number of genetic reads within the one corresponding group having the most abundant nucleotide *man_p* at the specific position *p* and the number of genetic reads within the one corresponding group is above or equal a predetermined threshold *t;* and

(ii) setting a tag *N* if the ratio *r* is below the predetermined threshold *t,*

wherein the one corresponding group is a group' and wherein a consensus sequence for a group" is created by performing steps (i) and (ii) for a further group.

In such aspects, the genetic reads of the group' may correspond to a first strand of a double-stranded nucleic acid and the genetic reads of the group" may correspond to the complementary second strand of said double-stranded nucleic acid.

The double-stranded consensus sequence may also be created, wherein the representation of the most abundant nucleotide *man_p* at the specific position *p* is set in the double-stranded consensus sequence if the representation is present in the consensus sequence for group' corresponding to the first strand and if the representation is present in the consensus sequence for the group" corresponding to the complementary second strand of said double-stranded nucleic acid.

In particular, the double-stranded consensus sequence may be created by

(i) setting a representation of the most abundant nucleotide *man_p* at each specific position *p* of a plurality of positions in the double strand consensus sequence if the representation or the tag at the specific position *p* is respectively present in both of the single strand consensus sequences of the group' and the group"; and

(ii) setting the tag "N" at each specific position *p* of a plurality of positions in the double strand consensus sequence if the tag "N" is present at the specific position *p* in one of the single strand consensus sequences of the group' or the group", or if the representation of the most abundant nucleotide *man_p* is not identical at the specific position in both of the single strand consensus sequences of the group' or the group".

The tag "N" at the specific position *p* can be set in the double strand consensus sequence

(i) if the tag N or the representation of the most abundant nucleotide *man_p* at the specific position *p* is present in the consensus sequence for the group' corresponding to the first strand and if the tag N or the representation of the most abundant nucleotide *man_p* at the specific position *p* is not present in the consensus sequence for the group" corresponding to the complementary second strand of said double-stranded nucleic acid; or

(ii) if the tag N at the specific position *p* is present in the consensus sequence for the group' corresponding to the first strand and if the tag N at the specific position *p* is present in the consensus sequence for the group" corresponding to the complementary second strand of said double-stranded nucleic acid.

Thus, the method may comprise grouping and the computation unit may be configured to group the corresponding single strand consensus sequences and collapsing the corresponding single strand consensus sequences to one double strand consensus sequence, wherein the representation of the most abundant nucleotide *man_p* is set or the tag "N" is set at the specific position *p* in the double strand consensus sequence if the representation or the tag at the specific position *p* is respectively present in both of the two corresponding single strand consensus sequences, or wherein the tag "N" is set at the specific position $p$ in the double strand consensus sequence if the tag "N" is present at the specific position $p$ in one of the two corresponding single strand consensus sequences.

In particular, the method may comprise grouping and the computation unit may be configured to group the corresponding single strand consensus sequences based on barcode sequences and collapsing the corresponding single strand consensus sequences to one double strand consensus sequence, wherein the representation of the most abundant nucleotide $man\_p$ is set or the tag "N" is set at the specific position $p$ in the double strand consensus sequence if the representation or the tag at the specific position $p$ is respectively present in both of the two corresponding single strand consensus sequences, or wherein the tag "N" is set at the specific position $p$ in the double strand consensus sequence if the tag "N" is present at the specific position $p$ in one of the two corresponding single strand consensus sequences.

Accordingly, the method may further comprise: creating a double-stranded consensus sequence by

(i) grouping the plurality of groups sharing the genetic position on a reference genetic sequence by a further particular feature(s), e.g., barcode sequence(s), wherein the plurality of groups comprise group' and group", wherein group' comprises genetic reads corresponding to the reverse complement of the genetic reads comprised in group",
(ii) creating a double-stranded consensus sequence by

(i) setting a representation of the most abundant nucleotide $man\_p$ or the tag "N" at each specific position $p$ of a plurality of positions in the double strand consensus sequence if the representation or the tag at the specific position $p$ is respectively present in both of the single strand consensus sequences of the group' and the group"; and
(ii) setting the tag "N" at each specific position $p$ of a plurality of positions in the double strand consensus sequence if the tag "N" is present at the specific position $p$ in one of the single strand consensus sequences of the group' or the group", or if the representation of the most abundant nucleotide $man\_p$ is not identical at the specific position in both of the single strand consensus sequences of the group' or the group".

The method of the invention is particularly suitable to identify (a) (genetic) variation(s) in the nucleic acid sequence of a subject.

In particular, in order to identify the variation(s), the method of the invention compares and the system of the invention is configured to compare the consensus sequences of the plurality of groups to the reference genetic sequence or the at least one reference genetic sequence at each specific position $p$ of a plurality of positions of the consensus sequences, and wherein a difference at a specific position between the consensus sequences of the plurality of groups and the reference genetic sequence or the at least one reference genetic sequence indicates a (genetic) variation at the specific position. This step is also exemplified in Figures 4 and 5.

In this context, the consensus sequences of the plurality of groups are compared to the reference genetic sequence, wherein the plurality of groups may refer to at least about 50%, preferably at least about 60%, more preferably at least about 70%, more preferably at least about 80%, more preferably at least about 90%, more preferably at least about 99%, or most preferably 100% of the groups which are formed by the grouping of the genetic reads sharing a genetic position on a reference genetic sequence.

As used herein, the term "comparing the consensus sequences of the plurality of groups to the reference genetic sequence at each specific position $p$ of a plurality of positions of the consensus sequences" or grammatical variants thereof means that each specific position nucleotide for nucleotide of at least about 50%, preferably at least about 60%, more preferably at least about 70%, more preferably at least about 80%, more preferably at least about 90%, more preferably at least about 99%, or most preferably 100% of the positions of the consensus sequences are compared to the reference genetic sequence.

In particular aspects, the consensus sequences of the plurality of groups are compared to the reference genetic sequence at each specific position $p$ of a plurality of positions of the consensus sequences, wherein all respective positions are compared and wherein a difference at a specific position between the consensus sequences and the reference genetic sequence indicates a (genetic) variation at the specific position.

In particular aspects, the consensus sequences of the plurality of groups are compared to the reference genetic sequence at each specific position $p$ of a plurality of positions of the consensus sequences, wherein all respective positions and wherein all consensus sequences are compared to the reference genetic sequence, and wherein a difference at a specific position between the consensus sequences and the reference genetic sequence indicates a genetic variation at the specific position.

As used herein, the term "comparing" or grammatical variants thereof in this context means that the consensus sequences are aligned to the reference genetic sequence. A difference at a specific position between the consensus sequences created and the reference genetic sequence indicates a (genetic) variation at specific position. In this step, the nucleotide (the representation of the nucleotides) of consensus sequences and the reference genetic sequence is compared. In particular, the (genetic) variation is not indicated at the specific position if the tag N is set at the specific position of the consensus sequence. Accordingly, a (genetic) variation is indicated at a specific position if a difference between a representation of the nucleotide set at the specific position in one corresponding consensus sequence and the nucleotide of the reference genetic sequence is comprised. Accordingly, the difference as used in this context does not refer to the tag N.

In particular aspects, the consensus sequences of the plurality of groups are compared to the reference genetic sequence at each specific position $p$ of a plurality of positions of the consensus sequences, and wherein a difference at a specific position between the most abundant nucleotide $man\_p$ of one corresponding consensus sequence and the reference genetic sequence indicates a variation at the specific position.

Accordingly, the method of the invention compares and the system of the invention is configured to compare the consensus sequences of the plurality of groups to the reference genetic sequence at each specific position p of a plurality of positions of the consensus sequences, and wherein a difference at a specific position between the most abundant nucleotide $man\_p$ of the consensus sequences and the representation of the reference genetic sequence indicates a genetic variation at the specific position.

In other words, the method of the invention compares and the system of the invention is configured to compare the consensus sequences of the plurality of groups to the reference genetic sequence at each specific position $p$ of a plurality of positions of the consensus sequences, if a difference at a specific position between the representation of the nucleotide(s) of the consensus sequences and the nucleotide of the reference genetic sequence is comprised.

In certain aspects, the consensus sequences of the second plurality of groups or of at least a further plurality of group can be compared to the reference genetic sequence.

Further, the method of the invention determines and the system of the invention is configured to determine the number of consensus sequences comprising the variation at each specific position $p$ of a plurality of positions, and method of the invention determines and the system of the invention is configured to determine the number of consensus sequences comprising the tag $N$ at each specific position $p$ of a plurality of positions. This step is also exemplified in Figures 4 and 5. As used herein, the term "determining the number of consensus sequences comprising the variation at each specific position $p$ of a plurality of positions" or grammatical variants thereof refers to determining the number of the consensus sequences of the plurality of groups. In this context the plurality of groups may refer to at least about 50%, preferably at least about 60%, more preferably at least about 70%, more preferably at least about 80%, more preferably at least about 90%, more preferably at least about 99%, or most preferably 100% of the groups which are formed by the grouping of the genetic reads sharing a genetic position on a reference genetic sequence. In particular, the plurality of groups (in the comparing step) may correspond to the plurality of groups queried in the comparing step. Accordingly, the consensus sequences of which the number is determined may refer to the consensus sequences of the plurality of groups that are compared to the reference genetic sequence.

In addition, as used herein, the term "the number of consensus sequences comprising the variation at each specific position $p$ of a plurality of positions" refers to the number of the consensus sequences that comprise the variation at each specific position $p$ of a plurality of positions. Accordingly, the number of variations at each specific position of at least about 50%, preferably at least about 60%, more preferably at least about 70%, more preferably at least about 80%, more preferably at least about 90%, more preferably at least about 99%, or most preferably 100% of the positions is determined.

In particular aspects, the number of consensus sequences comprising the variation at each specific position $p$ of a plurality of positions is determined, wherein the number of the variations of all respective positions is determined.

In particular aspects, the number of consensus sequences comprising the variation at each specific position $p$ of a plurality of positions is determined, wherein the number of the variations of all consensus sequences of the plurality of groups is determined.

In particular aspects, the number of consensus sequences comprising the variation at each specific position $p$ of a plurality of positions is determined, wherein the number of the variations of all respective positions and all consensus sequences of the plurality of groups are determined.

In certain aspects, the number of the consensus sequences of the second plurality of groups or of at least a further plurality of groups comprising the variation at each specific position $p$ of a plurality of positions can be determined, and the number of the consensus sequences of the second plurality of groups or of at least a further plurality of groups comprising the tag $N$ at each specific position $p$ of a plurality of positions can be determined.

Further, the method of the invention determines and the system of the invention is configured to determine the number of consensus sequences comprising the tag $N$ at each specific position $p$ of a plurality of positions. The above provided definitions and explanations also apply mutatis mutandis to the determination of the number of consensus sequences comprising N.

Further, the method of the invention identifies and the system of the invention is configured to identify the (genetic) variation at each specific position $p$ of a plurality of positions as a true (genetic) variation if a ratio $r^*$ between the number of consensus sequences comprising the tag $N$ at the specific position $p$ and the number of the consensus sequences comprising the (genetic) variation at the specific position $p$ is below a threshold $t^*$.

Accordingly, this step uses the determined numbers of the consensus sequences comprising the (genetic) variation or the tag N at each specific position $p$ of a plurality of positions to decide whether the (genetic) variation is a true or a false genetic variation. This step is also exemplified in Figures 4 and 5.

The "variation" refers herein to a variation in a nucleotide that occurs at a specific position in the genome, wherein such a nucleotide does not occur in a homologous or corresponding position in the at least one reference genetic sequence. The variation can also be understood as mutation, single nucleotide polymorphism (SNP) or variant allele. The variations can include a substitution(s), deletion(s) or addition(s) at any position in the nucleic acid sequence compared to the at least one reference genetic sequence. The term "variation" can mean a "genetic variation".

As used herein, the term "true variation" refers to a variation determined in the consensus sequences that fulfills the criteria of the N-filter. Accordingly, the variation determined in the consensus sequence is a true variation if a ratio $r^*$ between the number of consensus sequences comprising the tag $N$ at the specific position $p$ and the number of the consensus sequences comprising the variation at the specific position $p$ is below a threshold $t^*$. Accordingly, the true variation has a reduced probability to be a false positive variation, e.g. a false mutation call. Thus, the true probability can have a high probability to be a true mutation call.

As used herein, the threshold "$t^*$" or "t*" refers to any number that is used as a cutoff for the ratio $r^*$ between the number of consensus sequences comprising the tag $N$ at the specific position $p$ and the number of the consensus sequences comprising the variation at the specific position $p$ is below a threshold $t^*$.

As used herein, the ratio "$r^*$" or "r*" refers to the ratio between the number of consensus sequences comprising the tag N at the specific position p and the number of the consensus sequences comprising the variation at the specific position p.

In certain aspects, the variation is identified at each specific position $p$ of a plurality of positions as a true variation, wherein the plurality of positions refers to at least about 50%, preferably at least about 60%, more preferably at least about 70%, more preferably at least about 80%, more preferably at least about 90%, more preferably at least about 99%, or most preferably 100% of positions of the consensus sequence (s) .

In particular aspects, the variation is identified as a true variation at each specific position $p$ of a plurality of positions of the consensus sequences of the plurality of the groups. In this context, the plurality of groups may refer to at least about 50%, preferably at least about 60%, more preferably at least about 70%, more preferably at least about 80%, more preferably at least about 90%, more preferably at least about 99%, or most preferably 100% of the groups which are formed by the grouping of the genetic reads. In particular, the plurality of groups (in the identifying step) may correspond to the plurality of groups queried in comparing step and/or determining step.

In particular aspects, identifying the variation at each specific position $p$ of a plurality of positions as a true variation, wherein the true variation is to be identified at all respective positions.

In particular aspects, identifying the variation at each specific position $p$ of a plurality of positions as a true variation, wherein the true variation is to be identified at all respective consensus sequences of the plurality of the groups.

In particular aspects, identifying the variation at each specific position $p$ of a plurality of positions as a true variation, wherein the true variation is to be identified at all respective positions and at all respective the consensus sequences of the plurality of the groups.

In certain aspects, the true variation of the consensus sequences of the second plurality of groups or of at least a further plurality of groups can be identified.

Further, the variation at each specific position $p$ of a plurality of positions may be identified as a true variation if a ratio $r^*$ between the number of consensus sequences comprising the tag $N$ at the specific position $p$ and the number of the consensus sequences comprising the variation at the specific position $p$ is equal or below about 1, 1.1, 1.2, 1.3, 1.4, 1.5, 1.6, 1.7, 1.8.

In particular aspects, the variation at each specific position $p$ of a plurality of positions is identified as a true variation if a ratio $r^*$ between the number of consensus sequences comprising the tag $N$ at the specific position $p$ and the number of the consensus sequences comprising the variation at the specific position $p$ is equal or below 1.8.

In particular, the ratio r is equal or above 76% and the ratio $r^*$ is equal or below 1.8.

In particular, the method and systems of the invention are particularly suitable to identify variations with an allele frequency of less than 5%, 4%, 3%, 2% or particularly 1%. The method and systems of the invention are particularly suitable to identify variations with an allele frequency of less than 0.01%, 0.05%, 0.1%, 0.2% or particularly 1%.

The number of the genetic reads covering a specific position of a plurality of positions is a consequence of the applied sequencing depth. Accordingly, the higher the sequencing depth the more reads cover a defined position. A sub fraction of these reads might contain a variant and another sub fraction might contain "N". The ratio of N and the variant at such position is used to determine the "N"-variant ratio which is used to filter our false positive mutation calls.

In certain aspects, the sequencing of the nucleic acid sample has a sequencing depth/coverage of 100-times to 50000-times. In certain aspects, the sequencing of the nucleic acid sample has a sequencing depth/coverage of at least 100-times to less than 50000-times.

The true variation(s) may also be identified by employing the double stranded consensus sequence. The invention relates to a method and system being configured for

(a) optionally obtaining a plurality of genetic reads by sequencing of a nucleic acid sample;
(b) aligning the plurality of genetic reads to at least one reference genetic sequence;
(c) grouping the genetic reads sharing a genetic position on a reference genetic sequence of the at least one reference genetic sequence into a plurality of groups;
(d) creating a consensus sequence for each group of the plurality of groups, wherein one corresponding consensus sequence is created by determining the most abundant nucleotide $man\_p$ at each specific position $p$ of a plurality of positions within the one corresponding group of genetic reads and

(i) setting a representation of the most abundant nucleotide $man\_p$ at each specific position $p$ of the consensus sequence if a ratio r between the number of genetic reads within the one corresponding group having the most abundant nucleotide $man\_p$ at the specific position $p$ and the number of genetic reads within the one corresponding group is above or equal a predetermined threshold $t$; and
(ii) setting a tag $N$ if the ratio $r$ is below the predetermined threshold $t$, wherein said threshold $t$ is at least about 76%, wherein the one corresponding group is a group' and wherein a consensus sequence for a group" is created by performing steps (i) and (ii) for a further group, and
creating a double-stranded consensus sequence by
(iii) setting a representation of the most abundant nucleotide $man\_p$ or the tag "N" at each specific position $p$ of a plurality of positions in the double strand consensus sequence if the representation or the tag at the specific position $p$ is respectively present in both of the single strand consensus sequences of the group' and the group"; and
(iv) setting the tag "N" at each specific position $p$ of a plurality of positions in the double strand consensus sequence if the tag "N" is present at the specific position $p$ in one of the single strand consensus sequences of the group' or the group", or if the representation of the most abundant nucleotide $man\_p$ is not identical at the specific position in both of the single strand consensus sequences of the group' or the group";

(e) comparing the double stranded consensus sequence(s) and/or the consensus sequences of the plurality of the groups to the reference genetic sequence at each specific position $p$ of a plurality of positions of the consensus sequences, and wherein a difference at a specific position between the consensus sequences and the reference genetic sequence indicates a variation at the specific position;
(f) determining the number of consensus sequences comprising the variation at each specific position $p$ of a plurality of positions, and determining the number of consensus sequences comprising the tag $N$ at each specific position $p$ of a plurality of positions; and
(g) identifying the variation at each specific position $p$ of a plurality of positions as a true variation if a ratio $r^*$ between the number of consensus sequences comprising the tag $N$ at the specific position $p$ and the number of the consensus sequences comprising the variation at the specific position $p$ is below a threshold $t^*$, wherein said threshold $t^*$ is equal or below 1.8.

As described above, amplification of the nucleic acids/nucleic acid molecules can be employed. Amplification is a method for generating large amounts of a target sequence. In general, one or more amplification primers are annealed to a nucleic acid sequence. Using appropriate enzymes, sequences found adjacent to or in between the primers are amplified. Prior to or concurrent with analysis, the sample may be amplified by a variety of mechanisms. In some aspects nucleic acid amplification methods such as PCR may be combined with the disclosed methods and systems. See, for example, PCR Technology: Principles and Applications for DNA Amplification (Ed. H.A. Erlich, Freeman Press, NY, NY, 1992); PCR Protocols: A Guide to Methods and Applications (Eds. Innis, et al., Academic Press, San Diego, CA, 1990); Mattila et al., Nucleic Acids Res. 19, 4967 (1991); Eckert et al., PCR Methods and Applications 1, 17 (1991); PCR (Eds. McPherson et al., IRL Press, Oxford).

As used herein, the "subject" (or "patient") may be a vertebrate. In the context of the present invention, the term "subject" includes both humans and animals, particularly mammals, and other organisms. Thus, the herein provided methods are applicable to both human and animal subjects. Accordingly, said subject may be an animal such as a mouse, rat, hamster, rabbit, guinea pig, ferret, cat, dog, chicken, sheep, bovine species, horse, camel, or primate. Preferably, the subject is a mammal. Most preferably, the subject is human. The method, systems and kits provided herein can be used on any subject that is a healthy subject or a subject that suffers from any disease or disorder. In preferred aspects, the subject suffers from a disease, disorder or medical condition.

The nucleic acid sample, the nucleic acid, or the nucleic acid molecule can for example be or can comprise DNA, RNA, amplicons, cDNA, dsDNA, ssDNA, plasmid DNA, cosmid DNA, high Molecular Weight (MW) DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch, DNA-RNA hybrid, and viral RNA (e.g., retroviral RNA).

As used herein, the term "sample" is a biological sample that is obtained from the subject. "Sample" as used herein may, e.g., refer to a sample of bodily fluid or tissue obtained for the purpose of diagnosis, prognosis, or evaluation of a subject of interest, such as a patient. Preferably herein, the sample is a sample of a bodily fluid, such as blood, serum, plasma, pleura, cerebrospinal fluid, urine, saliva, sputum, and pleural effusions. Particularly, the sample is blood, blood plasma, blood serum, or urine. The sample may be pre-treated, e.g. by purification procedures, for example, separation of whole blood into serum or plasma components. Such pre-treatments can also include, but are not limited to dilution, filtration, centrifugation, concentration, sedimentation, precipitation or dialysis. Pre-treatments may also include the addition of chemical or biochemical substances to the solution, such as acids, bases, buffers, salts, solvents, reactive dyes, detergents, emulsifiers, chelators.

The term "nucleic acid sample" refers to a sample that comprises one or more nucleic acids or one or more nucleic acid molecules. The sample may thus be processed in order to obtain a nucleic acid sample. Accordingly, after obtaining a sample, e.g., a plasma sample, the nucleic acids or nucleic acid molecules may be isolated and/or extracted from the sample to obtain a nucleic acid sample. The nucleic acids or nucleic acid molecules may be isolated/extracted from the sample by any physical and chemical methods known to the skilled person. For example, the nucleic acids can be extracted from a sample by phenol-chloroform extraction, differential precipitation, ethanol precipitation, in-gel separation or solid-phase separation.

Further clean up steps may be used such as silica based columns to remove contaminants or salts. General steps may be optimized for specific applications. Non specific bulk carrier polynucleotides, for example, may be added throughout the reaction to optimize certain aspects of the procedure such as yield. Isolation and purification of nucleic acid molecules may be accomplished using any means, including, but not limited to, the use of commercial kits and protocols provided by companies such as Sigma Aldrich, Life Technologies, Promega, Affymetrix, IBI or the like. Kits and protocols may also be non-commercially available. After isolation, in some cases, the nucleic acid sample may be pre-mixed with one or more additional materials, such as one or more reagents (e.g., ligase, protease, polymerase) prior to sequencing.

The nucleic acid molecule(s) or nucleic acid(s) of which the nucleic acid sequence is determined and that occur in the nucleic acid sample can be subjected to fragmentation in order to obtain a length of the nucleic acid molecules that is suitable for nucleic acid sequencing. Nucleic acid molecules that are longer than 1000 nucleotides are usually fragmented. The nucleic acid sample may comprise nucleic acid molecules with a length of less than 1000 nucleotides.

The nucleic acid molecules to be sequenced may be obtained/isolated from a biological sample first. Such nucleic acid molecules may then be fragmented to an optimal length. The optimal length may be determined by the downstream platform. The skilled person is aware of techniques to fragment the nucleic acid obtained from a sample. Exemplary techniques are acoustic shearing, applying of nebulization forces, soni-cation, needle shearing, french pressure and/or enzyme-based treatments by the simultaneous cleavage of both strands, or by generation of nicks on each strand of dsDNA to produce dsDNA breaks.

The present invention also relates to a computer program product comprising one or more computer readable media having computer executable for instructions for performing the steps of any one of the methods provided herein.

The invention further relates to a system that can be employed in the methods provided herein. In particular, the present invention relates to a system for nucleic acid sequencing, particularly for reducing the number of false-positives in nucleic acid sequencing. The herein provided definitions and explanations in relation to the methods and the steps of the methods (e.g. obtaining the plurality of genetic reads, aligning, grouping and creating the consensus sequence) also apply mutatis mutandis to the system of the invention. Accordingly, the herein provided system is configured to be employed in any step of the herein provided methods. For example, the herein provided system comprises a computation unit being configured to create a consensus sequence.

For example, the computation unit may be configured to align the plurality of genetic reads to at least one reference genetic sequence.

Further, the computation unit may be configured to group the genetic reads sharing a genetic position on a reference genetic sequence of the at least one reference genetic sequence into a plurality of groups.

Further, the computation unit being configured to create a consensus sequence for each group of the plurality of groups, wherein one corresponding consensus sequence is created by determining a most abundant nucleotide $man\_p$ at each specific position $p$ of a plurality of positions within the one corresponding group of genetic reads and

(i) setting a representation of the most abundant nucleotide $man\_p$ at each specific position $p$ of the consensus sequence if a ratio $r$ between the number of genetic reads within the one corresponding group having the most abundant nucleotide $man\_p$ at the specific position $p$ and the number of genetic reads within the one corresponding group is above or equal a predetermined threshold $t$; and

(ii) setting a tag $N$ if the ratio is below the predetermined threshold $t$.

Further, the computation unit being configured to compare the consensus sequences of the plurality of groups to the reference genetic sequence at each specific position $p$ of a plurality of positions of the consensus sequences, and wherein a difference at a specific position between the consensus sequences and the reference genetic sequence indicates a variation at the specific position.

Further, the computation unit being configured to determine the number of consensus sequences comprising the variation at each specific position $p$ of a plurality of positions, and determining the number of consensus sequences comprising the tag $N$ at each specific position $p$ of a plurality of positions.

Further, the computation unit being configured to identify the variation at each specific position $p$ of a plurality of positions as a true variation if a ratio $r*$ between the number of consensus sequences comprising the tag $N$ at the specific position $p$ and the number of the consensus sequences comprising the variation at the specific position $p$ is below a threshold $t*$.

Further, the computation unit can be configured to create a consensus sequence at each specific position p of a plurality of positions within the group of genetic reads, wherein a representation of a nucleotide at one specific position of the plurality of positions is set in the consensus sequence, if a ratio between the number of this nucleotide at this specific position and the number of the genetic reads within this group is above or equal a predetermined threshold t, and wherein a tag N is set in the consensus sequence at this specific position if the ratio is below the predetermined threshold t.

Further, the computation unit can be configured to create a consensus sequence at each specific position p of a plurality of positions within the group of genetic reads, wherein the nucleotide(s) are determined at each of the specific position of the plurality of positions, and

wherein a representation of a nucleotide of the nucleotide(s) at one specific position of the plurality of positions is set in the consensus sequence, if a ratio between the number of this nucleotide at this specific position and the number of the genetic reads within this group is above or equal a predetermined threshold t, and wherein a tag N is set in the consensus sequence at this specific position if the ratio is below the predetermined threshold t.

The computation unit may further be configured to align the plurality of genetic reads to at least one reference genetic sequence and/or to group the genetic reads before the consensus sequence is created by the computation unit. In particular, the computation unit is configured to group the genetic reads belonging to the same reference genetic sequence into a corresponding group. Before the consensus sequence is created by the computation unit, the computation unit may also be configured to align the plurality of genetic reads to at least one reference genetic sequence and/or to group the genetic reads into one or more groups, wherein the genetic reads comprised in one corresponding group share at least one particular feature, such as at least one particular nucleic acid sequence, e.g., at least one barcode sequence.

Further to the computation unit the system may comprise an obtaining unit to obtain the plurality of genetic reads. Preferably, the system further comprises an obtaining unit being configured to obtain the plurality of genetic reads for a nucleic acid to be sequenced. Further, the system may comprise an obtaining unit being configured to obtain the plurality of genetic reads by nucleic acid sequencing of a nucleic acid sample. The system may comprise an obtaining unit being configured to determine the nucleic acid sequence of the tagged amplicons by nucleic acid sequencing and thereby determining a plurality of the genetic reads.

The computation unit may be configured to create respective consensus sequences and is configured to set a respective representation or a respective tag N for all respective positions within the one ore more groups.

Preferably, the computation unit is configured to create a respective consensus sequence and is configured to set a respective representation or a respective tag $N$ for all respective positions within the group. Further, computation unit is configured to create a respective consensus sequence of a second group or at least one further group.

As described above such a second group may correspond to a group comprising the genetic reads of the complementary strand.

The invention further relates to kits, the use of the kits, e.g. in the methods as provided herein above. The kits may comprise the system comprising a computation unit and the computer program as provided above. The kit may further comprise detection reagents to determine the nucleic acid sequence of the nucleic acid sample and thereby determining the plurality of the genetic reads. For example, NextSeq 500/550 v2 Kits (Illumina), MiSeq Reagent Kit v3 (Illumina), TruSeq Exome (Illumina) or SureSelectXT Reagent Kits (Agilent Technologies) .

Such detection reagents may include adaptors as described above.

It is understood by the skilled person that the present invention is suitable for reducing the number of false-positives in a single strand and considering a single strand in the present invention is sufficient.

Nevertheless, the present invention also gives the opportunity to exploit (i) using the particular features, e.g. barcode sequences, to discriminate polymerase chain reaction (PCR) duplicates from biological replicates (such as individual DNA molecules) and/or (ii) the complementary nature of the two strands of a DNA molecule. Therefore, first, by identifying PCR duplicates, consensus sequences can be formed by discarding sequence information below a pre-defined abundance within the pool of PCR duplicates of the same nucleic acid molecule. Second, true mutations are expected to be present on both nucleic acid strands, whereas variants erroneously introduced during the sequencing workflow appear only on one nucleic acid strand.

The systems, kits and methods of the invention may also be used in the diagnosis, monitoring, and/or prognosis of disease and/or disorder. Several variations are known that may indicate that a subject suffers or is at risk of suffering from (a) disease(s) and/or disorder(s). Such variations could thus be employed in the diagnosis, monitoring and/or prognosis.

Such a disease and/or disorder that may be selected from the group consisting of a single-gene disorder, autosomal dominant disorder, autosomal recessive disorder, X-linked dominant disorder, X-linked recessive disorder, Y-linked disorder, mitochondrial disease and a genetic disorder associated with multiple genes.

The systems, kits and methods of the invention may also be used in the diagnosis, monitoring, and/or prognosis of cancer and/or tumorous disease(s). Particularly, the systems, kits and methods of the invention may also be used in the diagnosis, monitoring, and/or prognosis of cancer and/or tumorous disease(s), wherein the variation(s) in the nucleic acid sequence are identified.

"Cancer", in accordance with the present invention, refers to a class of diseases or disorders characterized by uncontrolled division of cells and the ability of these to spread, either by direct growth into adjacent tissue through invasion, or by implantation into distant sites by metastasis, where cancer cells are transported through the bloodstream or lymphatic system.

The "tumorous disease" can be any form of a cancer, a tumor or is chosen from pancreas cancer, breast cancer, epithelial cancer, hepatocellular carcinoma, cholangiocellular cancer, stomach cancer, colon cancer, prostate cancer, bladder cancer, tongue cancer, head and neck cancer, skin cancer (melanoma), a cancer of the urogenital tract, e.g., ovarian cancer, endometrial cancer, cervix cancer, and kidney cancer; lung cancer, gastric cancer, a cancer of the small intestine, liver cancer, gall bladder cancer, a cancer of the bile duct, esophagus cancer, a cancer of the salivary glands or a cancer of the thyroid gland.

The systems, kits and methods of the invention may also be used in the diagnosis, monitoring, and/or prognosis of cancer and/or (a) tumorous disease(s), wherein the variation(s) in the nucleic acid sequence are identified.

For example, blood from a subject at risk for cancer may be obtained and prepared to obtain a nucleic acid sample. The systems and methods herein provided may be employed to identify variations(s) or rare variation(s)/mutation(s) that may exist in certain cancers. The method may help to detect the presence of cancerous cells in the body, despite the absence of symptoms or other hallmarks of disease.

The methods, kits and systems herein provided may also be used in the early detection of a disease and/or disorder, e.g., cancer. The system and methods provided herein may be used to detect any number of variation(s) that may cause or result from cancers. These may include but are not limited to mutations, rare mutations, indels, copy number variations, transversions, translocations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, abnormal changes in nucleic acid methylation infection and cancer.

The systems, kits and methods provided herein may also be used to characterize certain cancers. Genetic data produced from the system and methods of this disclosure may allow practitioners to help better characterize a specific form of cancer. Often times, cancers are heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer.

The systems, kits and methods provided herein may be used to monitor already diagnosed disease(s) and/or disorder(s). This may allow either a subject or practitioner to adapt treatment options in accordance with the progress of the disease and/or disorder. For example, the systems and methods described herein may be used to construct genetic profiles of a particular subject of the course of the disease and/or disorder. In some instances, cancers can progress, become more aggressive and genetically unstable. In other examples, cancers may remain benign, inactive, dormant or in remission. The system and methods of this disclosure may be useful in determining disease and/or disorder progression, remission or recurrence.

Further, the systems, kits and methods provided herein may be useful in determining the efficacy of a particular treatment option. In one example, successful treatment options may actually increase or decrease the amount of (a) variation(s) or rare mutation(s) identified in nucleic acid sample in response to the treatment. For example, if the treatment is successful, more cancerous and/or tumorous cells may die and shed DNA. In other examples, this may not occur. In another example, perhaps certain treatment options may be correlated with genetic profiles of cancers over time. This correlation may be useful in selecting a therapy. Additionally, if a cancer is observed to be in remission after treatment, the systems, kits and methods described herein may be useful in monitoring residual disease and/or disorder or recurrence of disease.

The methods, kits and systems provided herein may not be limited to detection of variation(s) associated with cancers. Various other diseases and infections may result in other types of conditions that may be suitable for early detection and monitoring. For example, in certain cases, genetic disorders or infectious diseases may cause a certain genetic mosaicism within a subject. This genetic mosaicism may cause copy number variation and variation(s) (e.g. rare mutations) that could be observed.

In another example, the system, kits and methods of the disclosure may also be used to monitor the genomes of immune cells within the body. Immune cells, such as cells, may undergo rapid clonal expansion upon the presence certain diseases. Clonal expansions may be monitored using copy number variation detection and certain immune states may be monitored.

Further, the systems, kits and methods provided herein may also be used to monitor systemic infections themselves, as may be caused by a pathogen such as a bacteria or virus. Variation(s) or copy number variation identification may be used to determine how a population of pathogens are changing during the course of infection. This may be particularly important during chronic infections, such as HTV/AIDs or Hepatitis infections, whereby viruses may change life cycle state and/or mutate into more virulent forms during the course of infection.

Yet another example that the system, kits and methods provided herein may be used for is the monitoring of transplant subjects. Generally, transplanted tissue undergoes a certain degree of rejection by the body upon transplantation.

The systems, kits and methods of the invention may also be used in the genotyping of a subject, wherein the variation(s) in the nucleic acid sequence of the subject are identified. As used herein, the term "genotyping" refers to determining difference(s) in the genotype of a subject by analyzing a nucleic acid sample of the subject and comparing it to another subject's sequence or at least one reference sequence. Genotyping may reveal the alleles the subject has inherited from their parents.

The systems, kits and methods of the invention may also be used to detect and count somatic alterations that may define neo-epitopes and may therefore initiate immune responses towards the tumor cells. Thus, sample specific mutational-load as well as identified neo-epitopes may function as biomarkers to stratify patients for treatment with immune checkpoint inhibitors.

The term "nucleotide" as described herein, refers to any and all nucleotide or any suitable natural or non-natural DNA or RNA nucleotide or nucleotide-like substance or analog with base pairing properties as described above

As used herein, the terms "comprising" and "including" or grammatical variants thereof are to be taken as specifying at least the stated features, integers, steps or components but do not preclude the addition of one or more additional features, integers, steps, components or groups thereof. This term encompasses the terms "consisting of" and "consisting essentially of" that are understood to specify only the stated feature, integers, steps or components to the exclusion of any additional features.

Thus, the terms "comprising"/"including"/"having" mean that any further component (or likewise features, integers, steps and the like) can/may be present.

The term "consisting of" means that no further component (or likewise features, integers, steps and the like) is present.

The term "consisting essentially of" or grammatical variants thereof when used herein are to be taken as specifying the stated features, integers, steps or components but do not preclude the addition of one or more additional features, integers, steps, components or groups thereof but only if the additional features, integers, steps, components or groups thereof do not materially alter the basic and novel characteristics of the claimed composition, device or method.

Thus, the term "consisting essentially of" means those specific further components (or likewise features, integers, steps and the like) can be present, namely those not materially affecting the essential characteristics of the composition, device or method. In other words, the term "consisting essentially of" (which can be interchangeably used herein with the term "comprising substantially"), allows the presence of other components in the composition, device or method in addition to the mandatory components (or likewise features, integers, steps and the like), provided that the essential characteristics of the device or method are not materially affected by the presence of other components.

The term "method" refers to manners, means, techniques and procedures for accomplishing a given task including, but not limited to, those manners, means, techniques and procedures either known to, or readily developed from known manners, means, techniques and procedures by practitioners of the chemical, biological and biophysical arts.

The term "about" preferably refers to $\pm 10\%$ of the indicated numerical value, more preferably to $\pm 5\%$ of the indicated numerical value, and in particular to the exact numerical value indicated.

As used herein, the term "about" refers to $\pm 10\%$ of the indicated numerical value, and in particular to $\pm 5\%$ of the indicated numerical value. Whenever the term "about" is used, a specific reference to the exact numerical value indicated is also included. If the term "about" is used in connection with a parameter that is quantified in integers, such as the number of nucleotides in a given nucleic acid, the numbers corresponding to $\pm 10\%$ or $\pm 5\%$ of the indicated numerical value are to be rounded to the nearest integer. For example, the expression "about 25 nucleotides" refers to the range of 23 to 28 nucleotides, in particular the range of 24 to 26 nucleotides, and preferably refers to the specific value of 25 nucleotides.

Unless otherwise indicated, established methods of recombinant gene technology were used as described, for example, in Sambrook, Russell "Molecular Cloning, A Laboratory Manual", Cold Spring Harbor Laboratory, N.Y. (2001))

# EP 3 409 788 B1

The present invention is further described by reference to the following non-limiting figures and examples.

## Brief description of the figures

Figure 1: (A) Schematic illustration of sheared DNA fragments labeled with double stranded predefined nucleotide sequences ("barcodes") (here represented with $\alpha$ and $\beta$). The number 1 and 2 shown in dark and light gray, respectively, represent the Illumina flow-cell-compatible tails. (B) Two different double stranded DNA molecules result from the PCR amplification of the barcoded DNA fragments shown in (A). Those resulting from the amplification of the upper strand have barcode $\alpha$ near the flow cell sequence number 1 and barcode $\beta$ near the flow cell sequence number 2 ("$\alpha\beta$ families"). PCR duplicates resulting from the amplification of the bottom strand have the two DNA complementary strands labeled reciprocally ("$\beta\alpha$ families"). Adapted from Kennedy SR et al. Nature Protocols (2014).

Figure 2: Schematic illustration of the computational analysis performed to generate synthetic single strand consensus (sscs) reads. Mutations/variations are highlighted by circles. Three different possible scenarios are here presented. Reads belonging to one family are compared at each genomic position and a nucleotide is written in to the sscs read only when at least 76% (4 reads out of 5) of the members of the family show the same base at the investigated position. If consensus is not reached, the assessed position in the sscs read is filled with an "N". Adapted from Schmitt MW et al. Proc Natl Acad Sci U S A. (2012).

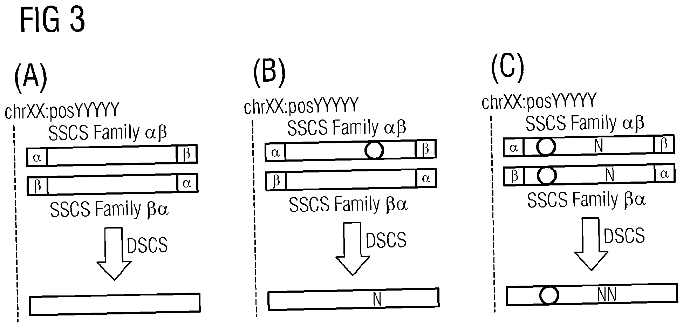

Figure 3: Schematic illustration of the computational analysis performed to generate synthetic double strand consensus (dscs) reads. Mutations/variations are highlighted by circles. scsc reads aligning at the same genomic location and characterized by reciprocal barcodes ($\alpha\beta$ and $\beta\alpha$ families) are compared. A nucleotide is written into a double strand consensus (dscs) read only if it is present in both sscs reads, otherwise the assessed position in the dscs read is filled with an "N". Addapted from Schmitt MW et al. Proc Natl Acad Sci U S A. (2012).

Figure 4: Example for a given position in which a nucleotide different from the nucleotide annotated in the reference genome is detected, if at that position more than twice as many "N" are present (compared to the variant), no mutation is called.

Figure 5: Example for a given position in which a nucleotide different from the nucleotide annotated in the reference genome is detected, if at that position more than four times as many "N" are present (compared to the variant), no mutation is called.

## Description of exemplary embodiments

Fig. 2 illustrates a method according to an exemplary embodiment of the present invention showing a specifically developed computational pipeline for the analysis of barcoded raw sequencing data.

In the inventive method as shown in Fig. 2, only the information of a single strand of a nucleic acid sequence is created, a so-called single strand consensus, hereinafter referred to as sscs. The first step of the computational flow of the method as presented in Fig. 2 is aligning the raw sequencing data to the human reference genome. In other words, the first step of the computational flow of the method is aligning a plurality of genetic reads to at least one reference genetic sequence. During this process, the first nucleotides of each read, representing the predefined barcode, are removed and placed into the read name to be available for the following step of the computational analysis.

After the alignment, read pairs sharing the same genomic position and barcodes are grouped into "families" as shown in Fig. 2. In other words, the second step refers to grouping the genetic reads belonging to the same reference genetic sequence into a corresponding group.

The aim is to generate a synthetic single strand consensus (sscs) read by using the information contained in all members of the family, i.e. by considering the most abundant sequencing information as true. Therefore, the reads belonging to the same family are compared nucleotide by nucleotide and the most abundant nucleotide *man_p* is determined at each position.

A respective most abundant nucleotide *man_p* is written into the sscs synthetic read at the respective position only if the nucleotide appears e.g. in at least in 76%, e.g. > 3 out of 4, of the members of the family at the investigate genomic position. In other words, the most abundant nucleotide *man_p* is determined at each specific position p of a plurality of positions within the respective family. A representation of the most abundant nucleotide *man_p* at each specific position p of the consensus sequence is only set if the ratio r between the number of nucleotides of the family at the specific position being the most abundant nucleotide *man_p* and the total number of reads of the family, i.e. the number of family members, is above or equal the predetermined threshold t, i.e. 76% in the present inventive embodiment. Otherwise, a tag N is set, i.e. if the ratio r between the number of nucleotides being the most abundant nucleotide *man_p* at the specific position p and the number of reads is below the 76%.

The upper part of Fig. 2A shows the situation in the $\alpha\beta$ family where only one read contains a variation/mutation (shown as circle) at an investigated position.

Such variation is only contained in one of the genetic reads. Thus, this nucleotide is not the most abundant nucleotide and the predetermined threshold, e.g. at least 76%, is not fulfilled. Therefore, the most abundant wild-type nucleotide (at least 76%) comprised in the other genetic reads at this position is set in the consensus sequence.

At the lower part of Fig. 2A, the respective $\beta\alpha$ family is shown. The members of the $\beta\alpha$ family do not show the variation as found in the upper family.

Fig. 2B shows the case where for the $\alpha\beta$ family a consensus for an investigate position can be found as the variation, which is the most abundant nucleotide *man_p*, occurs in every read in the family. Therefore, a representation for the most abundant nucleotide *man_p*, which is the variant, is set at the investigate position. In contrast, the respective $\beta\alpha$ family does not show said most abundant nucleotide *man_p* for the $\alpha\beta$ family at all.

Fig. 2C shows the case where for the $\alpha\beta$ family a consensus at a certain investigate position can be found but other positions do not fulfill the requirement that the respective nucleotide has to be present at the investigate position in e.g. 76% of the members of the family. Therefore, an "N" tag is said for the cases where none of the respective nucleotides is present at the respective investigate position in $\geq$ 76% of the members of the family.

Fig. 3 illustrates a method according to a preferred exemplary embodiment of the present invention.

After the single strand consensus sequence (sscs) has been determined as shown in Fig. 2, the sscs reads aligning at the same genomic location and characterized by reciprocal barcodes, i.e. the $\alpha\beta$ and $\beta\alpha$ families, are compared.

A nucleotide is written into a double strand consensus read only if this nucleotide is the most abundant nucleotide *man_p* in both sscs reads, otherwise the assessed position in the the sscs read is set with an "N" tag.

Fig. 3A shows the situation where the sscs of the both strands, i.e. $\alpha\beta$ family and $\beta\alpha$ family, agree with each other. Therefore, the dscs sequence is identical to the sequence of both sscs reads and no representation/tag is set in the double strand consensus.

Fig. 3B shows the situation where a certain most abundant nucleotide *man_p* can be found only in one of the families, i.e. the $\alpha\beta$ family, but not in the $\beta\alpha$ family. Therefore, as this nucleotide is only present in one of the families but not in the reciprocal family, the tag "N" is set at the double strand consensus.

In Fig. 3C, a certain most abundant nucleotide *man_p* is found for the $\alpha\beta$ family at a certain position p but also for the reciprocal $\beta\alpha$ family at the same respective position *p*. Therefore, a representation of said most abundant nucleotide *man_p* is written in the double strand consensus (circle). In contrast, at two distinct other positions, a "N" tag was set in either the $\alpha\beta$ family or the $\beta\alpha$ family. Because the "N" tag was only set in either one of the reciprocal families, a respective tag "N" is set at the double strand consensus at the respective position.

Examples

To assess the sensitivity and specificity (PPV) of the method according to the present invention in detecting variants at low MAF (minor allele frequency $\leq$ 1%), four dilutions of three HapMap normal cell lines are generated. Details can be taken from Table 1.

DNA from these four dilutions is analyzed using the method of creating a single strand consensus as described above.

**Table 1: Details of the HapMap normal cell lines dilutions generated in the laboratory in order to assess the sensitivity and specificity of our approach in detecting variants at low MAF**

| HapMap Normal Name | Dilution 1 | Dilution2 | Dilution 3 | Dilution 4 |
|---|---|---|---|---|
|  |  |  |  |  |
| GM19194B (%) | 99.4 | 99.4 | 99.4 | 99.4 |
| GM19153B (%) | 0.2 | 0.4 | na | na |
| GM12144C (%) | 0.4 | 0.2 | na | na |
| GM19137B (%) | na | na | 0.2 | 0.4 |
| GM19142B (%) | na | na | 0.4 | 0.2 |

A set of single nucleotide polymorphisms, SNPs specific to the individual HapMap cell lines in use, are determined from each non-diluted cell line. These were sub-divided into a set of unique SNPs, hereinafter referred to as "private SNPs", which are specific to one of the HapMap cell lines in use. SNPs present in more than one of the cell lines in use is referred to as non-private SNP. The sum of private and non-private SNPs is referred to as total SNPs.

HapMap cell line dilutions are used to determine the limit of detection of our assay. Because only heterozygous private SNPs were considered, all dilutions were characterized by private SNPs with expected 0.1%=<MAF<0.2% and MAF ∼ 50% as can be seen in table 1.

Due to experimental error, the detected MAF is in some cases different from that expected. Taking in consideration this intrinsic experimental error, sensitivity and specificity (PPV) were calculated as follow:

```
Sensitivity = True positive private SNPs/(True positive pri-
vate SNPs + False negative)
```

```
Specificity (PPV) = Total true positive/(Total true posi-
tive+False positive)
```

Where true positive, false negative and false positive are defines as:

- True positive private SNPs: private SNPs with detected 0.1% =< MAF < 0.2%
- True positive not private SNPs: not private SNPs with detected 0.1% =< MAF < 0.2%
- Total true positive: True positive private SNPs + True positive not private SNPs
- False negative: private SNPs characterized by a detected 0.1% =< MAF < 0.2% in the aligned sequencing read raw data but not called by our analysis algorithm.
- False positive: SNV with detected 0.1% =< MAF < 0.2% classified as "true mutations" by our analysis algorithm but not present in any of the non-diluted HapMap normal cell lines.

In Table 2 the details of the number of true positive, false negative and false positive with detected 0.1% =< MAF < 0.2% are reported for the four dilutions of HapMap normal cell lines presented in Table 1 at a mean sequencing coverage of 3000x.

**Table 2: Number true positive, false negative, false positive SNPs detected using the method presented in this document.**

| | Mean Coverage | Number of false negative private SNPs | Number of false positive variants | Number of true positive private SNPs | Total true positive SNPs |
|---|---|---|---|---|---|
| **Dilution 1** | 2943.56 | 6 | 16 | 25 | 91 |
| **Dilution 2** | 3042.1 | 2 | 12 | 27 | 49 |
| **Dilution 3** | 2995.51 | 4 | 18 | 30 | 58 |
| **Dilution 4** | 2934.2 | 4 | 25 | 34 | 66 |
| | | | | | |
| | total | 22 | 172 | 141 | 326 |

In the present invention a filter is developed that uses the level of reliability for each nucleotide call, thus exploits the nature of the sscs and/or dscs reads. As mentioned above, sscs reads are made of only those nucleotides present in at least 76% of the reads of a family at the investigated position.

If this condition is not fulfilled, an "N" is placed at the investigated position in the sscs read to indicate that the consensus was not reached. It is understood by the skilled person that the present invention works also only in case where only a single strand consensus sscs is considered.

However, in order to further improve the method of the present invention also the double strand consensus was considered. Therefore, similarly, a nucleotide is written into the synthetic dscs read only if present in both sscs reads aligning at the same genomic position and showing complementary barcodes, an "N" is placed at the investigated position in the dscs read if the consensus is not reached. Thus, positions with "N" represent regions for which a consensus was not reached and the true nature of the sequence is unknown.

It was observed that sscs and dscs reads aligning at genomic regions containing substitution erroneously introduced during the sequencing workflow exhibit a lower rate of consensus nucleotides and therefore a higher number of N compared to regions containing true variants. These regions may be defined by sequence repeats, which frequently lead to the incorporation of incorrect nucleotides and are therefore not removed by the 76% consensus cutoff applied in the previous step.

Based on this information, the abundance of "N" at a variant position across all reads covering the respective position (representing the sequencing depth at this position) can be used to discriminate a true mutation from erroneous substitution. In particular, the approach that has been implemented according to the present invention employs the ratio between the number of reads containing "N" and those containing the variant to discern a true call from false positive calls (referred to as "N filter" hereinafter).

### Example 1:

To assess the validity of the present invention, data obtained from the dilution of HapMap normal cell lines previously analyzed with the computational pipeline as used before the invention were further processed using the N filter with a required ratio of # (number) of reads containing a "N" at a defined position divided by the # (number) of reads containing the variant at the defined position is >2. Thus, if at a defined position more than twice as many "N" are present (compared to the variant), no mutation is called; see also Figure 4.

The number of true positive, false negative and false positive calls detected at 0.1% =< MAF < 0.2% using this further improved analysis is presented in Table 3.

**Table 3: Number true positive, false negative, false positive SNPs detected using the optimized computational pipeline including the N filter.**

| | Mean Coverage | Number of false negative private SNPs | Number of false positive variants | Number of true positive private SNPs | Total true (private + non-private SNPs) positive SNPs |
|---|---|---|---|---|---|
| **Dilution 1** | 2943.56 | 10 | 9 | 21 | 42 |
| **Dilution 2** | 3042.1 | 5 | 9 | 24 | 35 |
| **Dilution 3** | 2995.51 | 4 | 10 | 30 | 54 |
| **Dilution 4** | 2934.2 | 4 | 18 | 34 | 60 |
| | | | | | |
| | total | 23 | 46 | 109 | 191 |

The sensitivity and specificity (PPV) estimated for the workflow presented in details above were 82.5% and 80%, respectively with and without the N filter with a ratio> 2 (see Table 5).

### Example 2:

To assess the validity of the present invention, data obtained from the dilution of HapMap normal cell lines previously analyzed with the computational pipeline as used before the invention were further processed using the N filter with a required ratio of # (number) of reads containing a "N" at a defined position divided by the # (number) of reads containing the variant at the defined position is >4. Thus, if at a defined position more than four times as many "N" are present (compared to the variant), no mutation is called; see Figure 5.

The number of true positive, false negative and false positive calls detected at 0.1% =< MAF < 0.2% using this further improved analysis is presented in Table 3.

**Table 4: Number true positive, false negative, false positive SNPs detected using the optimized computational pipeline including the N filter.**

| | Mean Coverage | Number of false negative private SNPs | Number of false positive variants | Number of true positive private SNPs | Total true (private + non-private SNPs) positive SNPs |
|---|---|---|---|---|---|
| Dilution 1 | 2943.56 | 6 | 15 | 25 | 47 |
| Dilution 2 | 3042.1 | 2 | 11 | 27 | 44 |
| Dilution 3 | 2995.51 | 4 | 14 | 30 | 54 |
| Dilution 4 | 2934.2 | 4 | 20 | 34 | 60 |
| | | | | | |
| | total | 16 | 60 | 116 | 205 |

The sensitivity and specificity (PPV) estimated for the workflow presented in details above were 82.5% and 87.9%, respectively with and without the N filter with a ratio> 4 (see Table 5).

**Table 5: Sensitivity (PPV) obtained for the 4 dilutions of HapMap normal cell lines analyzed using the computational pipeline with two different N-filter ratios as well as without the N filter**

| | Sensitivity | Specificity (PPV) |
|---|---|---|
| without N filter | 86.5% | 65.5% |
| with N filter >4 | 87.9% | 77.4% |
| with N filter >2 | 82.5% | 80.5% |

Thus the present examples show using the above-described N filter shows a significant reduction in false-positives.

As the present invention may be embodied in several forms without departing from the scope or essential characteristics thereof, it should be understood that the above-described embodiments are not limited by any of the details of the foregoing descriptions, unless otherwise specified, but rather should be construed broadly within the scope as defined in the appended claims, and therefore all changes and modifications that fall within the present invention are therefore intended to be embraced by the appended claims.

References

[1] - Schmitt MW et al. Proc Natl Acad Sci U S A. (2012) and Kennedy SR et al. Nature Protocols (2014)

[2]-http://web.archive.org/web/20151107034222/http:/www.illumina.         com/documents/products/techspotlights/techspotlight_sequencin g.pdf

**Claims**

1. Method for nucleic acid sequencing comprising the following steps:

   (a) obtaining a plurality of genetic reads by sequencing of a nucleic acid sample;
   (b) aligning the plurality of genetic reads to at least one reference genetic sequence;
   (c) grouping the genetic reads sharing a genetic position on a reference genetic sequence of the at least one reference genetic sequence into a plurality of groups;
   (d) creating a consensus sequence for each group of the plurality of groups, wherein one corresponding consensus sequence is created by determining a most abundant nucleotide *man_p* at each specific position p of a plurality of positions within the one corresponding group of genetic reads and

(i) setting a representation of the most abundant nucleotide *man_p* at each specific position *p* of the consensus sequence if a ratio *r* between the number of genetic reads within the one corresponding group having the most abundant nucleotide *man_p* at the specific position *p* and the number of genetic reads within the one corresponding group is above or equal a predetermined threshold *t*; and

(ii) setting a tag *N* if the ratio *r* is below the predetermined threshold *t*, wherein said threshold *t* is at least about 76%;

(e) comparing the consensus sequences of the plurality of groups to the reference genetic sequence at each specific position *p* of a plurality of positions of the consensus sequences, and wherein a difference at a specific position between the consensus sequences and the reference genetic sequence indicates a genetic variation at the specific position;

(f) determining the number of consensus sequences comprising the variation at each specific position *p* of a plurality of positions, and determining the number of consensus sequences comprising the tag *N* at each specific position *p* of a plurality of positions; and

(g) identifying the genetic variation at each specific position *p* of a plurality of positions as a true genetic variation if a ratio *r\** between the number of consensus sequences comprising the tag *N* at the specific position *p* and the number of the consensus sequences comprising the genetic variation at the specific position *p* is below a threshold *t\**, wherein said threshold *t\** is equal or below 1.8.

2.  Method according to claim 1, wherein in step (c) each genetic read in a corresponding group of the plurality of groups comprises at least one particular nucleic acid sequence, such as at least one barcode sequence.

3.  Method according to claim 2, wherein each particular nucleic acid sequence, such as each barcode sequence, corresponds to a respective molecule.

4.  Method according to any one of the preceding claims, wherein the genetic reads of step (c) are grouped based on their genetic position and their barcode sequence.

5.  Method according to any one of the preceding claims, wherein in step (d) one corresponding group of the plurality of groups share at least one particular nucleic acid sequence, such as at least one barcode sequence.

6.  Method according to any one of the preceding claims, wherein step (d) is performed for all respective positions within the group, wherein step (e) is performed for all respective positions within the group, wherein step (f) is performed for all respective positions within the group, and/or wherein step (g) is performed for all respective positions within the group.

7.  Method according to any of the preceding claims, wherein the number of positions in the genetic reads is 72.

8.  Method according to any one of the preceding claims, wherein one corresponding group comprises at least 3 genetic reads.

9.  Method according to any one of the preceding claims, wherein the plurality of groups is at least two groups.

10. Method according to any one of the preceding claims, wherein the plurality of groups comprises a group' and group", and wherein the genetic reads of the group" at least partially overlap with the genetic reads of the group'.

11. Method according to any one of claims 1 to 10, wherein the plurality of groups comprises a group' and group", and wherein the genetic reads of the group" do not overlap with the genetic reads of the group'.

12. Method according to any one of claims 1 to 10, wherein the plurality of groups comprises a group' and group", and wherein the genetic reads of the group" fully overlap with the genetic reads of the group'.

13. Method according to any one of claims 1 to 10, wherein the plurality of groups comprises a group' and group", and wherein the genetic reads of the group" correspond to the reverse complement of the genetic reads of the group'.

14. Method according to claim 13, wherein the genetic reads of the group' correspond to a first strand of a double-stranded nucleic acid and the genetic reads of the group" correspond to the complementary second strand of said double-stranded nucleic acid.

15. Method according to claim 13 or 14, wherein a single strand consensus sequence is created for the group' and wherein a single strand consensus sequence is created for the group''.

16. Method according to any one of the preceding claims, further comprising:
creating a double-stranded consensus sequence by

(i) setting a representation of the most abundant nucleotide *man_p* or the tag "N" at each specific position *p* of a plurality of positions in the double strand consensus sequence if the representation or the tag at the specific position *p* is respectively present in both of the single strand consensus sequences of the group' and the group"; and
(ii) setting the tag "N" at each specific position *p* of a plurality of positions in the double strand consensus sequence if the tag "N" is present at the specific position *p* in one of the single strand consensus sequences of the group' or the group", or if the representation of the most abundant nucleotide *man_p* is not identical at the specific position in both of the single strand consensus sequences of the group' or the group".

17. Method according to claim 16, wherein in step (e) of claim 1 double-stranded consensus sequences are compared.

18. Method according to claim 17, wherein steps (e), (f), and (g) of claim 1 are performed with the double-stranded consensus sequences.

19. Method according to any one of claims 14 to 18, wherein each position corresponds to a base pair.

20. Method according to any of the preceding claims, wherein the genetic reads of the one corresponding group have the same length.

21. Method according to any one of the preceding claims, wherein the sequencing is next generation sequencing.

22. System for nucleic acid sequencing comprising:

(a) an obtaining unit being configured to obtain a plurality of genetic reads by sequencing of a nucleic acid sample; and
(b) a computation unit being configured to align the plurality of genetic reads to at least one reference genetic sequence;
(c) the computation unit being configured to group the genetic reads sharing a genetic position on a reference genetic sequence of the at least one reference genetic sequence into a plurality of groups;
(d) the computation unit being configured to create a consensus sequence for each group of the plurality of groups, wherein one corresponding consensus sequence is created by determining a most abundant nucleotide *man_p* at each specific position *p* of a plurality of positions within the one corresponding group of genetic reads and

(i) setting a representation of the most abundant nucleotide *man_p* at each specific position *p* of the consensus sequence if a ratio r between the number of genetic reads within the one corresponding group having the most abundant nucleotide *man_p* at the specific position *p* and the number of genetic reads within the one corresponding group is above or equal a predetermined threshold *t*; and
(ii) setting a tag *N* if the ratio is below the predetermined threshold *t*, wherein said threshold t is at least about 76%;

(e) the computation unit being configured to compare the consensus sequences of the plurality of groups to the reference genetic sequence at each specific position *p* of a plurality of positions of the consensus sequences, and wherein a difference at a specific position between the consensus sequences and the reference genetic sequence indicates a variation at the specific position;
(f) the computation unit being configured to determine the number of consensus sequences comprising the variation at each specific position *p* of a plurality of positions, and determining the number of consensus sequences comprising the tag *N* at each specific position *p* of a plurality of positions; and
(g) the computation unit being configured to identify the variation at each specific position *p* of a plurality of positions as a true variation if a ratio $r^*$ between the number of consensus sequences comprising the tag *N* at the specific position *p* and the number of the consensus sequences comprising the variation at the specific position *p* is below a threshold $t^*$, wherein said threshold $t^*$ is equal or below 1.8.

23. The system according to claim 22, wherein in step (c) each genetic read in a corresponding group of the plurality of groups comprises at least one particular nucleic acid sequence, such as at least one barcode sequence.

24. The system according to any one of claims 22 to 23, wherein each particular nucleic acid sequence, such as each barcode sequence, corresponds to a respective molecule.

25. The system according to any one of claims 22 to 24, wherein the genetic reads of step (c) are grouped based on their genetic position and their barcode sequence.

26. The system according to any one of claims 22 to 23, wherein in step (d) one corresponding group of the plurality of groups share at least one particular nucleic acid sequence, such as at least one barcode sequence.

27. The system according to any one of claims 22 to 26, wherein the computation unit being configured to create the consensus sequences and is configured to set a respective representation or a respective tag N for all respective positions within the one corresponding group, wherein the computation unit being configured to compare the consensus sequences to the reference genetic sequence at all positions, wherein the computation unit being configured to determine the number of consensus sequences comprising the variation and to determine the number of consensus sequences comprising the tag $N$ for all respective positions of the consensus sequences, and/or wherein the computation unit being configured to identify the variation at all positions and is configured to set a respective representation or a respective tag N for all respective positions of the consensus sequences.

28. The system according to any one of claims 22 to 27, wherein the number of positions in the genetic reads is 72.

29. The system according to any one of claims 22 to 28, wherein one corresponding group comprises at least 3 genetic reads.

30. The system according to any one of claims 22 to 29, wherein the plurality of groups is at least two groups.

31. The system according to any one of claims 22 to 30, wherein the plurality of groups comprises a group' and group", and wherein the genetic reads of the group" at least partially overlap with the genetic reads of the group'.

32. The system according to any one of claims 22 to 30, wherein the plurality of groups comprises a group' and group", and wherein the genetic reads of the group" do not overlap with the genetic reads of the group'.

33. The system according to any one of claims 22 to 30, wherein the plurality of groups comprises a group' and group", and wherein the genetic reads of the group" fully overlap with the genetic reads of the group'.

34. The system according to any one of claims 22 to 30, wherein the plurality of groups comprises a group' and group", and wherein the genetic reads of the group" correspond to the reverse complement of the genetic reads of the group'.

35. The system according to claim 34, wherein the genetic reads of the group' correspond to a first strand of a double-stranded nucleic acid and the genetic reads of the group" correspond to the complementary second strand of said double-stranded nucleic acid.

36. The system according to claim 34 or 35, wherein a single strand consensus sequence is created for the group' and wherein a single strand consensus sequence is created for the group''.

37. The system according to any one of claims 22 to 36, wherein the computation unit being configured to create a double-stranded consensus sequence by

(i) setting a representation of the most abundant nucleotide $man\_p$ or the tag "N" at each specific position p of a plurality of positions in the double strand consensus sequence if the representation or the tag at the specific position $p$ is respectively present in both of the single strand consensus sequences of the group' and the group"; and

(ii) setting the tag "N" at each specific position $p$ of a plurality of positions in the double strand consensus sequence if the tag "N" is present at the specific position $p$ in one of the single strand consensus sequences of the group' or the group", or if the representation of the most abundant nucleotide $man\_p$ is not identical at the specific position in both of the single strand consensus sequences of the group' or the group".

**38.** The system according to claim 37, wherein the computation unit being configured to compare double-stranded consensus sequences.

**39.** The system according to claim 37 or 38, wherein the computation unit being configured to compare the double-stranded consensus sequences, to determine the number of the double-stranded consensus sequences, and to identify the variation in double-stranded consensus sequences.

**40.** The system according to any one of claims 22 to 39, wherein each position corresponds to a base pair.

**41.** The system according to any one of claims 22 to 40, wherein the genetic reads of the one corresponding group have the same length.

**42.** The system according to any one of claims 22 to 41, wherein the sequencing is next generation sequencing.

**43.** Computer program product comprising one or more computer readable media having computer executable instructions for performing the steps of the method of any one of claims 1 to 21.


**Patentansprüche**

**1.** Verfahren zur Nukleinsäuresequenzierung, das die folgenden Schritte umfasst:

(a) den Erhalt mehrerer genetischer Abschnitte durch das Sequenzieren einer Nukleinsäureprobe;
(b) das Alignment der mehreren genetischen Abschnitte mit wenigstens einer genetischen Referenzsequenz;
(c) das Gruppieren der genetischen Abschnitte, die eine genetische Position auf einer genetischen Referenzsequenz der wenigstens einen genetischen Referenzsequenz gemeinsam aufweisen, zu mehreren Gruppen;
(d) das Erzeugen einer Konsensussequenz für jede Gruppe der mehreren Gruppen, wobei eine entsprechende Konsensussequenz erzeugt wird, indem ein häufigstes Nukleotid $man\_p$ an jeder spezifischen Position $p$ mehrerer Positionen innerhalb der einen entsprechenden Gruppe von genetischen Abschnitten bestimmt wird, und

(i) das Festlegen einer Darstellung des häufigsten Nukleotids $man\_p$ an jeder spezifischen Position $p$ der Konsensussequenz, wenn ein Verhältnis $r$ zwischen der Zahl von genetischen Abschnitten innerhalb der einen entsprechenden Gruppe mit dem häufigsten Nukleotid $man\_p$ an der spezifischen Position $p$ und der Zahl von genetischen Abschnitten innerhalb der einen entsprechenden Gruppe größer als ein oder gleich einem vorbestimmten Grenzwert $t$ ist; und
(ii) das Festlegen eines Tags $N$, wenn das Verhältnis $r$ kleiner als der vorbestimmte Grenzwert $t$ ist, wobei der Grenzwert t wenigstens etwa 76 % beträgt;

(e) das Vergleichen der Konsensussequenzen der mehreren Gruppen mit der genetischen Referenzsequenz an jeder spezifischen Position $p$ mehrerer Positionen der Konsensussequenzen, und wobei ein Unterschied an einer spezifischen Position zwischen den Konsensussequenzen und der genetischen Referenzsequenz auf eine genetische Variation an der spezifischen Position hindeutet;
(f) das Bestimmen der Zahl von Konsensussequenzen, welche die Variation an jeder spezifischen Position $p$ mehrerer Positionen umfassen, und das Bestimmen der Zahl von Konsensussequenzen, welche das Tag $N$ an jeder spezifischen Position $p$ mehrerer Positionen umfassen; und
(g) das Identifizieren der genetischen Variation an jeder spezifischen Position $p$ mehrerer Positionen als wahre genetische Variation, wenn ein Verhältnis $r*$ zwischen der Zahl von Konsensussequenzen, welche das Tag $N$ an der spezifischen Position $p$ umfassen, und der Zahl der Konsensussequenzen, welche die genetische Variation an der spezifischen Position p umfassen, kleiner als ein Grenzwert $t*$ ist, wobei der Grenzwert $t*$ gleich oder kleiner als 1,8 ist.

**2.** Verfahren nach Anspruch 1, wobei in Schritt (c) jeder genetische Abschnitt in einer entsprechenden Gruppe der mehreren Gruppen wenigstens eine bestimmte Nukleinsäuresequenz, wie wenigstens eine Barcode-Sequenz, umfasst.

**3.** Verfahren nach Anspruch 2, wobei jede bestimmte Nukleinsäuresequenz, wie jede Barcode-Sequenz, einem jeweiligen Molekül entspricht.

**4.** Verfahren nach einem der vorhergehenden Ansprüche, wobei die genetischen Abschnitte von Schritt (c) basierend auf ihrer genetischen Position und ihrer Barcode-Sequenz gruppiert werden.

**5.** Verfahren nach einem der vorhergehenden Ansprüche, wobei in Schritt (d) eine entsprechende Gruppe der mehreren Gruppen wenigstens eine bestimmte Nukleinsäuresequenz, wie wenigstens eine Barcode-Sequenz, gemeinsam aufweist.

**6.** Verfahren nach einem der vorhergehenden Ansprüche, wobei Schritt (d) für alle jeweiligen Positionen innerhalb der Gruppe durchgeführt wird, wobei Schritt (e) für alle jeweiligen Positionen innerhalb der Gruppe durchgeführt wird, wobei Schritt (f) für alle jeweiligen Positionen innerhalb der Gruppe durchgeführt wird und/oder wobei Schritt (g) für alle jeweiligen Positionen innerhalb der Gruppe durchgeführt wird.

**7.** Verfahren nach einem der vorstehenden Ansprüche, wobei die Zahl von Positionen in den genetischen Abschnitten 72 beträgt.

**8.** Verfahren nach einem der vorhergehenden Ansprüche, wobei eine entsprechende Gruppe wenigstens 3 genetische Abschnitte umfasst.

**9.** Verfahren nach einem der vorhergehenden Ansprüche, wobei die mehreren Gruppen wenigstens zwei Gruppen sind.

**10.** Verfahren nach einem der vorhergehenden Ansprüche, wobei die mehreren Gruppen eine Gruppe' und eine Gruppe'' umfassen und wobei die genetischen Abschnitte der Gruppe'' wenigstens teilweise mit den genetischen Abschnitten der Gruppe' überlappen.

**11.** Verfahren nach einem der Ansprüche 1 bis 10, wobei die mehreren Gruppen eine Gruppe' und eine Gruppe'' umfassen und wobei die genetischen Abschnitte der Gruppe'' nicht mit den genetischen Abschnitten der Gruppe' überlappen.

**12.** Verfahren nach einem der Ansprüche 1 bis 10, wobei die mehreren Gruppen eine Gruppe' und eine Gruppe'' umfassen und wobei die genetischen Abschnitte der Gruppe'' vollständig mit den genetischen Abschnitten der Gruppe' überlappen.

**13.** Verfahren nach einem der Ansprüche 1 bis 10, wobei die mehreren Gruppen eine Gruppe' und eine Gruppe'' umfassen und wobei die genetischen Abschnitte der Gruppe'' dem reversen Komplement der genetischen Abschnitte der Gruppe' entsprechen.

**14.** Verfahren nach Anspruch 13, wobei die genetischen Abschnitte der Gruppe' einem ersten Strang einer doppelsträngigen Nukleinsäure entsprechen und die genetischen Abschnitte der Gruppe'' dem komplementären zweiten Strang der doppelsträngigen Nukleinsäure entsprechen.

**15.** Verfahren nach Anspruch 13 oder 14, wobei eine Einzelstrang-Konsensussequenz für die Gruppe' erzeugt wird und wobei eine Einzelstrang-Konsensussequenz für die Gruppe'' erzeugt wird.

**16.** Verfahren nach einem der vorherigen Ansprüche, das weiterhin Folgendes umfasst:
das Erzeugen einer Doppelstrang-Konsensussequenz durch

(i) das Festlegen einer Darstellung des häufigsten Nukleotids *man_p* oder des Tags *"N"* an jeder spezifischen Position $p$ mehrerer Positionen in der Doppelstrang-Konsensussequenz, wenn die Darstellung oder das Tag an der spezifischen Position $p$ jeweils in beiden Einzelstrang-Konsensussequenzen der Gruppe' und der Gruppe'' vorhanden ist; und
(ii) das Festlegen des Tags "N" an jeder spezifischen Position $p$ mehrerer Positionen in der Doppelstrang-Konsensussequenz, wenn das Tag "N" an der spezifischen Position $p$ in einer der Einzelstrang-Konsensussequenzen der Gruppe' oder der Gruppe'' vorhanden ist oder wenn die Darstellung des häufigsten Nukleotids *man_p* mit der spezifischen Position in beiden Einzelstrang-Konsensussequenzen der Gruppe' oder der Gruppe'' nicht identisch ist.

**17.** Verfahren nach Anspruch 16, wobei in Schritt (e) von Anspruch 1 Doppelstrang-Konsensussequenzen verglichen werden.

**18.** Verfahren nach Anspruch 17, wobei die Schritte (e), (f) und (g) von Anspruch 1 mit den Doppelstrang-Konsensussequenzen durchgeführt werden.

**19.** Verfahren nach einem der Ansprüche 14 bis 18, wobei jede Position einem Basenpaar entspricht.

**20.** Verfahren nach einem der vorhergehenden Ansprüche, wobei die genetischen Abschnitte der einen entsprechenden Gruppe dieselbe Länge aufweisen.

**21.** Verfahren nach einem der vorhergehenden Ansprüche, wobei es sich bei der Sequenzierung um eine Next-Generation-Sequenzierung handelt.

**22.** System zur Nukleinsäuresequenzierung, umfassend:

(a) eine Erlangungseinheit, die dazu eingerichtet ist, mehrere genetische Abschnitte durch das Sequenzieren einer Nukleinsäureprobe zu erhalten; und
(b) eine Berechnungseinheit, die dazu eingerichtet ist, die mehreren genetischen Abschnitte mit wenigstens einer genetischen Referenzsequenz zu alignieren;
(c) wobei die Berechnungseinheit dazu eingerichtet ist, die genetischen Abschnitte, die eine genetische Position auf einer genetischen Referenzsequenz der wenigstens einen genetischen Referenzsequenz gemeinsam aufweisen, zu mehreren Gruppen zu gruppieren;
(d) die Berechnungseinheit dazu eingerichtet ist, eine Konsensussequenz für jede Gruppe der mehreren Gruppen zu erzeugen, wobei eine entsprechende Konsensussequenz erzeugt wird, indem ein häufigstes Nukleotid *man_p* an jeder spezifischen Position *p* mehrerer Positionen innerhalb der einen entsprechenden Gruppe von genetischen Abschnitten bestimmt wird, und

(i) das Festlegen einer Darstellung des häufigsten Nukleotids *man_p* an jeder spezifischen Position *p* der Konsensussequenz, wenn ein Verhältnis *r* zwischen der Zahl von genetischen Abschnitten innerhalb der einen entsprechenden Gruppe mit dem häufigsten Nukleotid *man_p* an der spezifischen Position *p* und der Zahl von genetischen Abschnitten innerhalb der einen entsprechenden Gruppe größer als ein oder gleich einem vorbestimmten Grenzwert *t* ist; und
(ii) das Festlegen eines Tags *N,* wenn das Verhältnis kleiner als der vorbestimmte Grenzwert t ist, wobei der Grenzwert t wenigstens etwa 76 % beträgt;

(e) die Berechnungseinheit dazu eingerichtet ist, die Konsensussequenzen der mehreren Gruppen mit der genetischen Referenzsequenz an jeder spezifischen Position *p* mehrerer Positionen der Konsensussequenzen zu vergleichen, und wobei ein Unterschied an einer spezifischen Position zwischen den Konsensussequenzen und der genetischen Referenzsequenz auf eine genetische Variation an der spezifischen Position hindeutet;
(f) die Berechnungseinheit dazu eingerichtet ist, die Zahl von Konsensussequenzen zu bestimmen, welche die Variation an jeder spezifischen Position *p* mehrerer Positionen umfassen, und die Zahl von Konsensussequenzen zu bestimmen, welche das Tag *N* an jeder spezifischen Position *p* mehrerer Positionen umfassen; und
(g) die Berechnungseinheit dazu eingerichtet ist, die Variation an jeder spezifischen Position *p* mehrerer Positionen als wahre Variation zu identifizieren, wenn ein Verhältnis *r\** zwischen der Zahl von Konsensussequenzen, welche das Tag *N* an der spezifischen Position *p* umfassen, und der Zahl der Konsensussequenzen, welche die Variation an der spezifischen Position *p* umfassen, kleiner als ein Grenzwert *t\** ist, wobei der Grenzwert *t\** gleich oder kleiner als 1,8 ist.

**23.** System nach Anspruch 22, wobei in Schritt (c) jeder genetische Abschnitt in einer entsprechenden Gruppe der mehreren Gruppen wenigstens eine bestimmte Nukleinsäuresequenz, wie wenigstens eine Barcode-Sequenz, umfasst.

**24.** System nach einem der Ansprüche 22 bis 23, wobei jede bestimmte Nukleinsäuresequenz, wie jede Barcode-Sequenz, einem jeweiligen Molekül entspricht.

**25.** System nach einem der Ansprüche 22 bis 24, wobei die genetischen Abschnitte von Schritt (c) basierend auf ihrer genetischen Position und ihrer Barcode-Sequenz gruppiert werden.

**26.** System nach einem der Ansprüche 22 bis 23, wobei in Schritt (d) eine entsprechende Gruppe der mehreren Gruppen wenigstens eine bestimmte Nukleinsäuresequenz, wie wenigstens eine Barcode-Sequenz, gemeinsam aufweist.

**27.** System nach einem der Ansprüche 22 bis 26, wobei die Berechnungseinheit dazu eingerichtet ist, die Konsenssequenzen zu erzeugen, und dazu eingerichtet ist, eine jeweilige Darstellung oder einen jeweiligen Tag N für alle jeweiligen Positionen innerhalb der einen entsprechenden Gruppe festzulegen, wobei die Berechnungseinheit dazu eingerichtet ist, die Konsenssequenzen an allen Positionen mit der genetischen Referenzsequenz zu vergleichen, wobei die Berechnungseinheit dazu eingerichtet ist, die Zahl von Konsenssequenzen zu bestimmen, welche die Variation umfassen, und die Zahl von Konsenssequenzen zu bestimmen, welche das Tag N an allen jeweiligen Positionen der Konsenssequenzen umfassen, und/oder wobei die Berechnungseinheit dazu eingerichtet ist, die Variation an allen Positionen zu identifizieren, und dazu eingerichtet ist, eine jeweilige Darstellung oder ein jeweiliges Tag N für alle jeweiligen Positionen der Konsenssequenzen festzulegen.

**28.** System nach einem der Ansprüche 22 bis 27, wobei die Zahl von Positionen in den genetischen Abschnitten 72 beträgt.

**29.** System nach einem der Ansprüche 22 bis 28, wobei eine entsprechende Gruppe wenigstens 3 genetische Abschnitte umfasst.

**30.** System nach einem der Ansprüche 22 bis 29, wobei die mehreren Gruppen wenigstens zwei Gruppen sind.

**31.** System nach einem der Ansprüche 22 bis 30, wobei die mehreren Gruppen eine Gruppe' und eine Gruppe'' umfassen und wobei die genetischen Abschnitte der Gruppe'' wenigstens teilweise mit den genetischen Abschnitten der Gruppe' überlappen.

**32.** System nach einem der Ansprüche 22 bis 30, wobei die mehreren Gruppen eine Gruppe' und eine Gruppe'' umfassen und wobei die genetischen Abschnitte der Gruppe'' nicht mit den genetischen Abschnitten der Gruppe' überlappen.

**33.** System nach einem der Ansprüche 22 bis 30, wobei die mehreren Gruppen eine Gruppe' und eine Gruppe'' umfassen und wobei die genetischen Abschnitte der Gruppe'' vollständig mit den genetischen Abschnitten der Gruppe' überlappen.

**34.** System nach einem der Ansprüche 22 bis 30, wobei die mehreren Gruppen eine Gruppe' und eine Gruppe'' umfassen und wobei die genetischen Abschnitte der Gruppe'' dem reversen Komplement der genetischen Abschnitte der Gruppe' entsprechen.

**35.** System nach Anspruch 34, wobei die genetischen Abschnitte der Gruppe' einem ersten Strang einer doppelsträngigen Nukleinsäure entsprechen und die genetischen Abschnitte der Gruppe'' dem komplementären zweiten Strang der doppelsträngigen Nukleinsäure entsprechen.

**36.** System nach Anspruch 34 oder 35, wobei eine Einzelstrang-Konsenssequenz für die Gruppe' erzeugt wird und wobei eine Einzelstrang-Konsenssequenz für die Gruppe'' erzeugt wird.

**37.** System nach einem der Ansprüche 22 bis 36, wobei die Berechnungseinheit dazu eingerichtet ist, eine Doppelstrang-Konsenssequenz zu erzeugen durch

(i) das Festlegen einer Darstellung des häufigsten Nukleotids $man\_p$ oder des Tags *"N"* an jeder spezifischen Position $p$ mehrerer Positionen in der Doppelstrang-Konsenssequenz, wenn die Darstellung oder das Tag an der spezifischen Position $p$ jeweils in beiden Einzelstrang-Konsenssequenzen der Gruppe' und der Gruppe'' vorhanden ist; und

(ii) das Festlegen des Tags "N" an jeder spezifischen Position $p$ mehrerer Positionen in der Doppelstrang-Konsenssequenz, wenn das Tag "N" an der spezifischen Position $p$ in einer der Einzelstrang-Konsenssequenzen der Gruppe' oder der Gruppe'' vorhanden ist oder wenn die Darstellung des häufigsten Nukleotids $man\_p$ mit der spezifischen Position in beiden Einzelstrang-Konsenssequenzen der Gruppe' oder der Gruppe'' nicht identisch ist.

**38.** System nach Anspruch 37, wobei die Berechnungseinheit dazu eingerichtet ist, Doppelstrang-Konsenssequenzen zu vergleichen.

**39.** System nach Anspruch 37 oder 38, wobei die Berechnungseinheit dazu eingerichtet ist, die Doppelstrang-Konsenssequenzen zu vergleichen, die Zahl der Doppelstrang-Konsenssequenzen zu bestimmen und die Variation in Doppelstrang-Konsensussequenzen zu identifizieren.

**40.** System nach einem der Ansprüche 22 bis 39, wobei jede Position einem Basenpaar entspricht.

**41.** System nach einem der Ansprüche 22 bis 40, wobei die genetischen Abschnitte der einen entsprechenden Gruppe dieselbe Länge aufweisen.

**42.** System nach einem der Ansprüche 22 bis 41, wobei es sich bei der Sequenzierung um eine Next-Generation-Sequenzierung handelt.

**43.** Computerprogrammprodukt, das ein oder mehrere computerlesbare Medien mit computerausführbaren Anweisungen zur Durchführung der Schritte des Verfahrens nach einem der Ansprüche 1 bis 21 umfasst.

**Revendications**

**1.** Méthode de séquençage d'acide nucléique, comprenant les stades suivants :

(a) obtenir une pluralité de lectures génétiques en séquençant un échantillon d'acide nucléique ;
(b) aligner la pluralité de lectures génétiques sur au moins une séquence génétique de référence ;
(c) regrouper les lectures génétiques, partageant une position génétique sur une séquence génétique de référence de la au moins une séquence génétique de référence, en une pluralité de groupes ;
(d) créer une séquence consensus pour chaque groupe de la pluralité de groupes, une séquence consensus correspondante étant créée en déterminant un nucléotide $man\_p$ le plus abondant à chaque position p spécifique d'une pluralité de positions au sein du un groupe correspondant de lectures génétiques et

(i) mettre une représentation du nucléotide $man\_p$ le plus abondant à chaque position p spécifique de la séquence consensus si un rapport r, entre le nombre de lectures génétiques au sein du un groupe correspondant ayant le nucléotide $man\_p$ le plus abondant à la position $p$ spécifique et le nombre de lectures génétiques au sein du un groupe correspondant, est supérieur ou égal à un seuil $t$ déterminé à l'avance ; et
(ii) mettre une étiquette $N$ si le rapport r est inférieur au seuil $t$ déterminé à l'avance, le seuil $t$ étant d'au moins environ 76% ;

(e) comparer les séquences consensus de la pluralité de groupes à la séquence génétique de référence, à chaque position $p$ spécifique d'une pluralité de positions des séquences consensus, et dans lequel une différence à une position spécifique entre les séquences consensus et la séquence génétique de référence indique une variation génétique à la position spécifique ;
(f) déterminer le nombre de séquences consensus comprenant la variation à chaque position $p$ spécifique d'une pluralité de positions et déterminer le nombre de séquences consensus comprenant l'étiquette $N$ à chaque position $p$ spécifique d'une pluralité de positions ; et
(g) identifier la variation génétique à chaque position $p$ spécifique d'une pluralité de positions, comme une variation génétique vraie
si un rapport $r^*$, entre le nombre de séquences consensus comprenant l'étiquette $N$ à la position $p$ spécifique et le nombre de séquences consensus comprenant la variation génétique à la position p spécifique, est inférieur à un seuil $t^*$, le seuil $t^*$ étant inférieur ou égal à 1,8.

**2.** Procédé suivant la revendication 1, dans lequel, dans le stade (c), chaque lecture génétique, dans un groupe correspondant de la pluralité de groupes, comprend au moins une séquence particulière d'acide nucléique, telle qu'au moins une séquence de code barre.

**3.** Procédé suivant la revendication 2, dans lequel chaque séquence particulière d'acide nucléique, telle qu'une séquence de code barre, correspond à une molécule respective.

**4.** Procédé suivant l'une quelconque des revendications précédentes, dans lequel on regroupe les lectures génétiques du stade (c) sur la base de leur position génétique et de leur séquence de code barre.

**5.** Procédé suivant l'une quelconque des revendications précédentes, dans lequel, dans le stade (d), un groupe correspondant de la pluralité de groupes partage au moins une séquence particulière d'acide nucléique, telle qu'au moins une séquence de code barre.

6. Procédé suivant l'une quelconque des revendications précédentes, dans lequel on effectue le stade (d) pour toutes les positions respectives au sein du groupe, le stade (e) étant effectué pour toutes les positions respectives au sein du groupe, le stade (f) étant effectué pour toutes les positions respectives au sein du groupe et/ou le stade (g) étant effectué pour toutes les positions respectives au sein du groupe.

7. Procédé suivant l'une quelconque des revendications précédentes, dans lequel le nombre de positions dans les lectures génétiques est de 72.

8. Procédé suivant l'une quelconque des revendications précédentes, dans lequel un groupe correspondant comprend au moins 3 lectures génétiques.

9. Procédé suivant l'une quelconque des revendications précédentes, dans lequel la pluralité de groupes est au moins deux groupes.

10. Procédé suivant l'une quelconque des revendications précédentes, dans lequel la pluralité de groupes comprend un groupe' et un groupe" et dans lequel les lectures génétiques du groupe" chevauchent, au moins en partie, les lectures génétiques du groupe'.

11. Procédé suivant l'une quelconque des revendications 1 à 10, dans lequel la pluralité de groupes comprend un groupe 'et un groupe", et dans lequel les lectures génétiques du groupe" ne chevauchent pas les lectures génétiques du groupe'.

12. Procédé suivant l'une quelconque des revendications 1 à 10, dans lequel la pluralité de groupes comprend un groupe' et un groupe" et dans lequel les lectures génétiques du groupe'' chevauchent complètement les lectures génétiques du groupe'.

13. Procédé suivant l'une quelconque des revendications 1 à 10, dans lequel la pluralité de groupes comprend un groupe' et un groupe" et dans lequel les lectures génétiques du groupe'' correspondent au complément inverse des lectures génétiques du groupe'.

14. Procédé suivant la revendication 13, dans lequel les lectures génétiques du groupe' correspondent à un premier brin d'un acide nucléique à double brin et les lectures génétiques du groupe" correspondent au deuxième brin complémentaire de l'acide nucléique à double brin.

15. Procédé suivant la revendication 13 ou 14, dans lequel on crée une séquence consensus à simple brin pour le groupe' et dans lequel on crée une séquence consensus à simple brin pour le groupe".

16. Procédé suivant l'une quelconque des revendications précédentes,
comprenant, en outre :
créer une séquence consensus à double brin en

(i) mettant une représentation du nucléotide *man_p* le plus abondant ou de l'étiquette « N » à chaque position p spécifique d'une pluralité de positions dans la séquence consensus à double brin si la représentation ou l'étiquette à la position p spécifique est présente, respectivement, à la fois dans les séquences consensus à simple brin du groupe' et du groupe" ; et
(ii) mettant l'étiquette « N » à chaque position p spécifique d'une pluralité de positions dans la séquence consensus à double brin si l'étiquette « N » est présente à la position p spécifique dans l'une des séquences consensus à simple brin du groupe' ou du groupe" ou si la représentation du nucléotide *man_p* le plus abondant n'est pas identique, à la position spécifique, à la fois des séquences consensus à simple brin du groupe' ou du groupe".

17. Procédé suivant la revendication 16, dans lequel, dans le stade (e) de la revendication 1, on compare des séquences consensus à double brin.

18. Procédé suivant la revendication 17, dans lequel on effectue les stades (e), (f) et (g) de la revendication 1 avec des séquences consensus à double brin.

**19.** Procédé suivant l'une quelconque des revendications 14 à 18, dans lequel chaque position correspond à une paire de bases.

**20.** Procédé suivant l'une quelconque des revendications précédentes, dans lequel les lectures génétiques du un groupe correspondant ont la même longueur.

**21.** Procédé suivant l'une quelconque des revendications précédentes, dans lequel le séquençage est un séquençage de génération immédiatement suivante.

**22.** Système de séquençage d'acide nucléique, comprenant :

(a) une unité d'obtention, configurée pour obtenir une pluralité de lectures génétiques en séquençant un échantillon d'acide nucléique ; et
(b) une unité informatique, configurée pour aligner la pluralité de lectures génétiques sur au moins une séquence génétique de référence ;
(c) l'unité informatique étant configurée pour regrouper des lectures génétiques, partageant une position génétique sur une séquence génétique de référence de la au moins une séquence génétique de référence, en une pluralité de groupes ;
(d) l'unité informatique étant configurée pour créer une séquence consensus pour chaque groupe de la pluralité de groupes, une séquence consensus correspondante étant créée en déterminant un nucléotide $man\_p$ le plus abondant à chaque position p spécifique d'une pluralité de positions au sein du un groupe correspondant de lectures génétiques et

(i) mettre une représentation du nucléotide $man\_p$ le plus abondant à chaque position p spécifique de la séquence consensus si un rapport r, entre le nombre de lectures génétiques au sein du un groupe correspondant ayant le nucléotide $man\_p$ le plus abondant à la position p spécifique et le nombre de lectures génétiques au sein du un groupe correspondant, est supérieur ou égal à un seuil t déterminé à l'avance ; et
(ii) mettre une étiquette $N$ si le rapport r est inférieur au seuil $t$ déterminé à l'avance, le seuil $t$ étant d'au moins environ 76% ;

(e) l'unité informatique étant configurée pour comparer les séquences consensus de la pluralité de groupes à la séquence génétique de référence à chaque position $p$ spécifique d'une pluralité de positions des séquences consensus, et dans lequel une différence à une position spécifique entre les séquences consensus et la séquence génétique de référence indique une variation à la position spécifique ;
(f) l'unité informatique étant configurée pour déterminer le nombre de séquences consensus comprenant la variation à chaque position $p$ spécifique d'une pluralité de positions et déterminer le nombre de séquences consensus comprenant l'étiquette $N$ à chaque position $p$ spécifique d'une pluralité de positions ; et
(g) l'unité informatique étant configurée pour identifier la variation à chaque position $p$ spécifique d'une pluralité de positions, comme une variation vraie si un rapport $r*$, entre le nombre de séquences consensus comprenant l'étiquette $N$ à la position $p$ spécifique et le nombre de séquences consensus comprenant la variation génétique à la position p spécifique, est inférieur à un seuil $t*$, le seuil $t*$ étant inférieur ou égal à 1,8.

**23.** Système suivant la revendication 22, dans lequel, dans le stade (c), chaque lecture génétique dans un groupe correspondant de la pluralité de groupes comprend au moins une séquence particulière d'acide nucléique, telle qu'au moins une séquence de code barre.

**24.** Système suivant l'une quelconque des revendications 22 à 23, dans lequel chaque séquence particulière d'acide nucléique, telle qu'une séquence de code barre, correspond à une molécule respective.

**25.** Système suivant l'une quelconque des revendications 22 à 24, dans lequel les lectures génétiques du stade (c) sont regroupées sur la base de leur position génétique et de leur séquence de code barre.

**26.** Système suivant l'une quelconque des revendications 22 à 23, dans lequel, dans le stade (d), un groupe correspondant de la pluralité de groupes partage au moins une séquence particulière d'acide nucléique, telle qu'au moins une séquence de code barre.

**27.** Système suivant l'une quelconque des revendications 22 à 26, dans lequel l'unité informatique est configurée pour créer les séquences consensus et est configurée pour mettre une représentation respective ou une étiquette N respective dans toutes les positions respectives au sein du un groupe correspondant, dans lequel l'unité informatique est configurée pour comparer les séquences consensus à la séquence génétique de référence à toutes les positions, dans lequel l'unité informatique est configurée pour déterminer le nombre de séquences consensus comprenant la variation et pour détermine le nombre de séquences consensus comprenant l'étiquette N pour toutes les positions respectives des séquences consensus et/ou dans lequel l'unité informatique est configurée pour identifier la variation à toutes les positions et est configurée pour mettre une représentation respective ou une étiquette N respective pour toutes les positions respectives des séquences consensus.

28. Système suivant l'une quelconque des revendications 22 à 27, dans lequel le nombre de positions dans les lectures génétiques est de 72.

29. Système suivant l'une quelconque des revendications 22 à 28, dans lequel un groupe correspondant comprend au moins 3 lectures génétiques.

30. Système suivant l'une quelconque des revendications 22 à 29, dans lequel la pluralité de groupes est au moins deux groupes.

31. Système suivant l'une quelconque des revendications 22 à 30, dans lequel la pluralité de groupe comprend un groupe' et un groupe" et dans lequel les lectures génétiques du groupe" chevauchent, au moins en partie, les lectures génétiques du groupe'.

32. Système suivant l'une quelconque des revendications 22 à 30, dans lequel la pluralité de groupes comprend un groupe' et un groupe", et dans lequel les lectures génétiques du groupe" ne chevauchent pas les lectures génétiques du groupe'.

33. Système suivant l'une quelconque des revendications 22 à 30, dans lequel la pluralité de groupes comprend un groupe' et un groupe" et dans lequel les lectures génétiques du groupe'' chevauchent complètement les lectures génétiques du groupe'.

34. Système suivant l'une quelconque des revendications 22 à 30, dans lequel la pluralité de groupes comprend un groupe' et un groupe" et dans lequel les lectures génétiques du groupe'' correspondent au complément inverse des lectures génétiques du groupe'.

35. Système suivant la revendication 34, dans lequel les lectures génétiques du groupe' correspondent à un premier brin d'un acide nucléique à double brin et les lectures génétiques du groupe" correspondent au deuxième brin complémentaire de l'acide nucléique à double brin.

36. Système suivant la revendication 34 ou 35, dans lequel une séquence consensus à simple brin est créée pour le groupe' et dans lequel une séquence consensus à simple brin est créée pour le groupe".

37. Système suivant l'une quelconque des revendications 22 à 36, dans lequel l'unité informatique est configurée pour créer une séquence consensus à double brin en

(i) mettant une représentation du nucléotide *man_p* le plus abondant ou de l'étiquette « N » à chaque position p spécifique d'une pluralité de positions dans la séquence consensus à double brin si la représentation ou l'étiquette à la position p spécifique est présente, respectivement, à la fois dans les séquences consensus à simple brin du groupe' et du groupe" ; et
(ii) mettant l'étiquette « N » à chaque position *p* spécifique d'une pluralité de positions dans la séquence consensus à double brin si l'étiquette « N » est présente à la position *p* spécifique dans l'une des séquences consensus à simple brin du groupe' ou du groupe" ou si la représentation du nucléotide *man_p* le plus abondant n'est pas identique à la position spécifique à la fois des séquences consensus à simple brin du groupe' ou du groupe".

38. Système suivant la revendication 37, dans lequel l'unité informatique est configurée pour comparer des séquences consensus à double brin.

39. Système suivant la revendication 37 ou 38, dans lequel l'unité informatique est configurée pour comparer les séquences consensus à double brin, pour déterminer le nombre de séquences consensus à double brin et pour identifier la variation dans des séquences consensus à double brin.

40. Système suivant l'une quelconque des revendications 22 à 39, dans lequel chaque position correspond à une paire de bases.

41. Système suivant l'une quelconque des revendications 22 à 40, dans lequel les lectures génétiques du un groupe correspondant ont la même longueur.

42. Système suivant l'une quelconque des revendications 22 à 41, dans lequel le séquençage est un séquençage de génération immédiatement suivante.

43. Produit de programme d'ordinateur comprenant un ou plusieurs supports déchiffrables par ordinateur, ayant des instructions exécutables par ordinateur pour effectuer les stades du procédé suivant l'une quelconque des revendications 1 à 21.

# FIG 1

## (A)

## (B)

αβ family

βα family

# FIG 2

## (A)

chrXX:posYYYYY

Family αβ

↓ SSCS

Family βα

↓ SSCS

## (B)

chrXX:posYYYYY

Family αβ

↓ SSCS

Family βα

↓ SSCS

## (C)

chrXX:posYYYYY

Family αβ

↓ SSCS

Family βα

↓ SSCS

# FIG 3

## (A)

chrXX:posYYYYY

SSCS Family αβ

α | | β
β | | α

SSCS Family βα

⬇ DSCS

## (B)

chrXX:posYYYYY

SSCS Family αβ

α | ◯ | β
β | | α

SSCS Family βα

⬇ DSCS

N

## (C)

chrXX:posYYYYY

SSCS Family αβ

α | ◯ | N | β
β | ◯ | N | α

SSCS Family βα

⬇ DSCS

◯ NN

FIG 4 — Example 1: for a given position in which a nucleotide different from the nucleotide annotated in the reference genome is detected, if at that position more than twice as many "N" are present (compared to the variant), no mutation is called.

Position of interest 1

Reference genome ⇒ ACTCCCTCGGATCATGACTAATCCAAATTTACACACACCTCTCGTCGCTGCTCGACAATATATCTATCTACAATGACATAGATATTACTG

Position of interest 2

Legend — ☐ Consensus read

Wilde type nucleotide = C
Variant nucleotide = T

#of reads with wilde type nucleotide = 2
#of reads with variant nucleotide = 4
#of reads with "N" = 10

$$\frac{\#\text{of reads with "N"}}{\#\text{of reads with variant nucleotide}} = \frac{10}{4} = 2.5 > 2$$

The variant nucleotide is filtered out and not called as true mutation

Wilde type nucleotide = T
Variant nucleotide = G

#of reads with wilde type nucleotide = 2
#of reads with variant nucleotide = 11
#of reads with "N" = 3

$$\frac{\#\text{of reads with "N"}}{\#\text{of reads with variant nucleotide}} = \frac{3}{11} = 0.45 < 2$$

The variant nucleotide is called as true mutation

FIG 5  Example 2: for a given position in which a nucleotide different from the nucleotide annotated in the reference genome is detected, if at that position more than four times as many "N" are present (compared to the variant), no mutation is called.

Position of interest 1

Reference genome ⇨ ACTCCCTCGGATCATGACTAATCCAAATTTACACACACCTCTCGTCGCTGCTCGACAATATATCTATCTACAATGACATAGATATTACTG

Position of interest 2

Legend

☐ Consensus read

Wilde type nucleotide = C
Variant nucleotide = T

#of reads with wilde type nucleotide = 2
#of reads with variant nucleotide = 1
#of reads with "N" = 13

$$\frac{\#of\ reads\ with\ "N"}{\#of\ reads\ with\ variant\ nucleotide} = \frac{13}{1} = 13 > 4$$

The variant nucleotide is filtered out and not called as true mutation

Wilde type nucleotide = T
Variant nucleotide = G

#of reads with wilde type nucleotide = 2
#of reads with variant nucleotide = 11
#of reads with "N" = 3

$$\frac{\#of\ reads\ with\ "N"}{\#of\ reads\ with\ variant\ nucleotide} = \frac{3}{11} = 0.27 < 4$$

The variant nucleotide is called as true mutation

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013142389 A
- US 61552374

**Non-patent literature cited in the description**

- **SHENDURE J ; JI H.** Next-generation DNA sequencing. *Nat Biotechnol,* 2008, vol. 26, 1135-1145
- **FOX EJ et al.** Accuracy of Next Generation Sequencing Platforms. *Next Generat Sequenc & Applic,* 2014, vol. 1, 106
- **NAKAMURA K.** Sequence-specific error profile of Illumina sequencers. *Nucleic Acids Res.,* July 2011, vol. 39 (13), e90
- **SCHIRMER M.** Illumina error profiles:resolving fine-scale variation in metagenomic sequencing data. *BMC Bioinformatics,* 11 March 2016, vol. 17, 125
- **KENNEDY et al.** *Nature Protocols,* 2014, vol. 9 (11
- **LI H. ; DURBIN R.** Fast and accurate short read alignment with Burrows-Wheeler Transform. *Bioinformatics,* 2009, vol. 25, 1754-60, http://bio-bwa.sourceforge.net/bwa.shtml
- PCR Technology: Principles and Applications for DNA Amplification. Freeman Press, 1992
- PCR Protocols: A Guide to Methods and Applications. Academic Press, 1990
- **MATTILA et al.** *Nucleic Acids Res.,* 1991, vol. 19, 4967
- **ECKERT et al.** *PCR Methods and Applications,* 1991, vol. 1, 17
- PCR. IRL Press
- **SAMBROOK ; RUSSELL.** Molecular Cloning, A Laboratory Manua. Cold Spring Harbor Laboratory, 2001
- **KENNEDY SR et al.** *Nature Protocols,* 2014
- **SCHMITT MW et al.** *Proc Natl Acad Sci U S A.,* 2012